# EUROPEAN PATENT APPLICATION

(11) **EP 4 218 787 A2**
(43) Date of publication of application: **02.08.2023**
(21) Application number: 23156938.5
(22) Date of filing: 14.08.2020
(51) Int. Cl.: A61K 38/08, A61P 21/00, A61P 25/00, A23L 33/18, C07K 7/00, C07K 14/415

(54) **PEPTIDES FOR TREATING MUSCLE ATROPHY**

(30) Priority: 20.08.2019 EP 19192689
(62) Divisional of application: 20767974.7
(71) Applicant: Nuritas Limited, D02 RY95 Dublin 2 (IE)
(72) Inventor: KHALDI, Nora, Dublin, D02 RY95 (IE); LOPEZ, Cyril, Dublin, D02 RY95 (IE); KHALDI, Nora, Dublin, D02 RY95 (IE)
(74) Representative: Purdylucey Intellectual Property

(57) **Abstract**

The Applicant has discovered a number of peptides that are capable of phosphorylating ribosomal protein S6 (rpS6) across a range of concentrations in in-vitro cell assays in a dose-dependent manner. RPS6 is a key substrate for protein kinases and is phosphorylated by growth factors and mitogens during in cell growth and cell division. This is a key step in the synthesis of new proteins in skeletal muscle tissue. The peptides described also have the ability to reduce the expression of mRNA Transcripts (TRIM63 and FBXO32) that are directly linked to increase protein degradation, resulting in progressive Skeletal Muscle Atrophy. In addition, increases in muscular atrophy are linked with systemic rise in circulating TNFα. The peptides described herein also lead to the reduced expression of TNFα in circulating immune cells. The peptides may be used to promote muscle growth and muscle health in subjects that exhibit muscle atrophy, for example elderly subjects, physically inactive people, and subjects that have indications characterised by muscle atrophy (i.e. MS and Polio).

## Description

### Field of the Invention

The present invention relates to a number of peptides, and compositions comprising one or more of the peptides. Also contemplated are the use of the peptide or compositions to treat or prevent muscle atrophy in subjects.

### Background to the Invention

Muscle atrophy is a condition in humans characterised by muscle wastage, increase in protein degradation, increased systemic inflammation, and a down-regulation of newly synthesised protein in the affected muscle types. Symptoms of the condition include marked weakness in one or more limbs, one arm or leg being noticeably smaller than the other. It is commonly associated with aging (often as part of sarcopaenia), with subjects who have been physically inactive for long periods, for example bedridden patients, astronauts, injured sportspeople, subjects with eating disorders, subjects with metabolic disease, and subjects who have diseases characterised by neuronal, muscle or joint degeneration such as ALS, MS, muscular dystrophy, Guillane-Barre syndrome, sarcopaenia, osteoarthritis, polio, rheumatoid arthritis, spinal muscular atrophy and polymyositis.

Ribosomal protein S6 is one of 33 proteins that, together with one molecule of 18 S rRNA, comprise the small 40 S ribosomal subunit (4). rpS6 directly interacts with the m7GpppG 5'-cap-binding complex required for translation initiation and represents a point of regulatory convergence for signal transduction pathways controlling translation initiation in response to cell growth and cell proliferation cues. rpS6 undergoes inducible phosphorylation in response to mitogenic and cell growth stimuli, and this phosphorylation is conserved in vertebrates, invertebrates, plants, and fungi (5). In higher eukaryotes, phosphorylation occurs on a cluster of five serine residues at the carboxyl terminus of rpS6: Ser-235, Ser-236, Ser-240, Ser-244, and Ser-247 (6). Drosophila rpS6 contains a similar organization of five phosphorylation sites, whereas the homolog found in Saccharomyces cerevisiae contains two Ser residues corresponding to mammalian Ser-235 and Ser-236 (4). Phosphorylation of rpS6 occurs in an ordered manner, beginning with Ser-236 and followed sequentially by phosphorylation of Ser-235, Ser-240, Ser-244, and Ser-247 (7, 8). The phosphorylation of rpS6 on C-terminal residues enhances its affinity for the m7GpppG cap, which strongly implies that rpS6 phosphorylation enhances mRNA translation initiation.

Carboxyl-terminal phosphorylation of rpS6 is regulated by at least two signal transduction pathways. The p70 ribosomal S6 kinases, S6K1 and S6K2, play a major role in rpS6 C terminus phosphorylation in response to insulin, serum, and amino acid stimulation (4). S6K1 and S6K2 phosphorylate Ser-240 and Ser-244 but are dispensable for Ser-235 and Ser-236 phosphorylation in intact cells (13). The activities of S6K1 and S6K2 are in turn directly regulated by the mammalian target of rapamycin, mTOR, which responds to growth and mitogenic cues. Inhibition of mTOR with rapamycin causes a drastic reduction in rpS6 phosphorylation in mammalian cells (14). mTOR also phosphorylates the translational repressor 4E-BP1, causing its dissociation from the m7GpppG 5'-cap-binding complex and, through combined phosphorylation of S6Ks and 4E-BP1, mTOR positively regulates protein translation in response to favorable growth conditions. The RAS/ERK pathway also regulates rpS6 phosphorylation independent of mTOR through the activation of p90 ribosomal S6K kinases, RSK1 and RSK2 (12). RSK1 and RSK2 phosphorylate rpS6 on Ser-235 and Ser-236 in response to phorbol ester, serum, and oncogenic RAS, and the phosphorylation of both residues is required for cap binding (13).

It is an object of the invention to overcome at least one of the above-referenced problems.

### Summary of the Invention

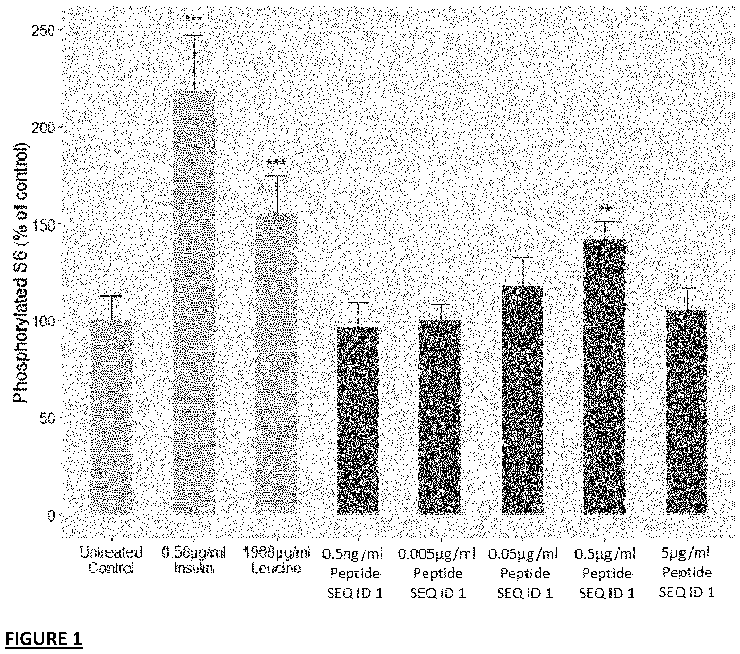

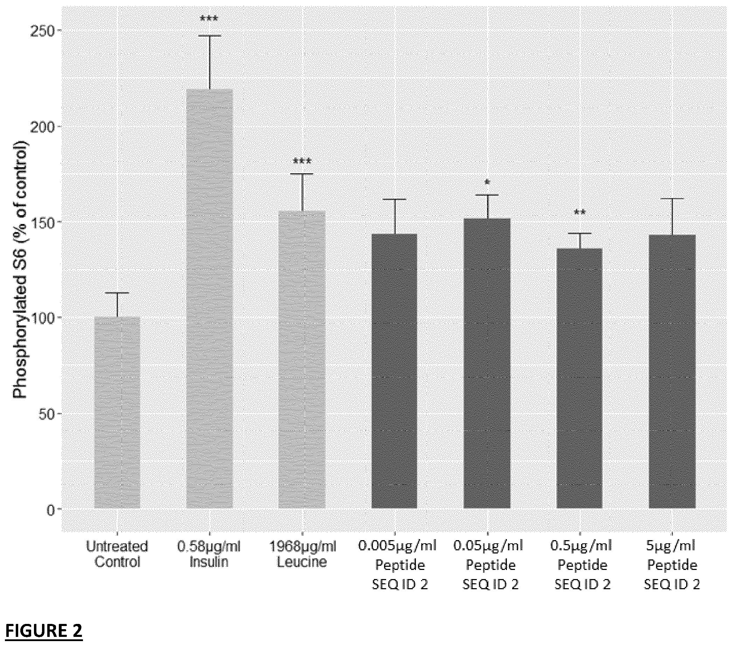

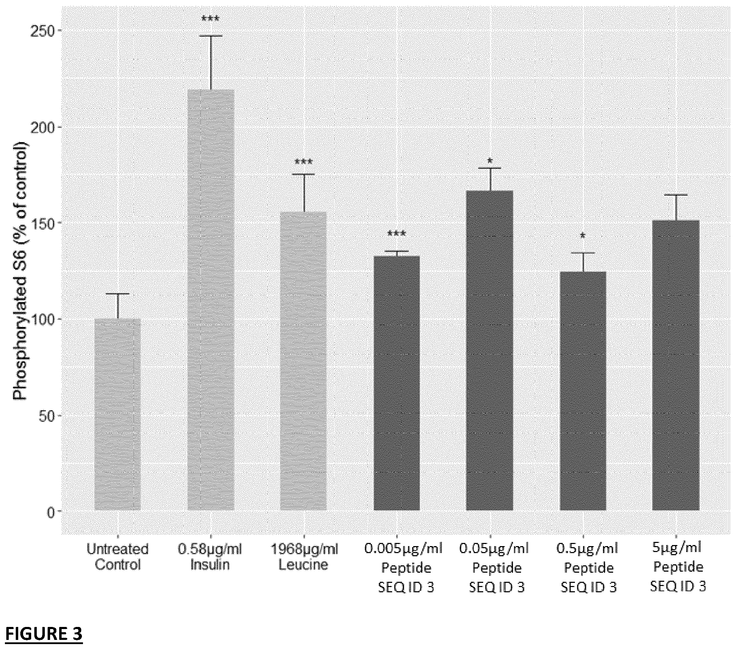

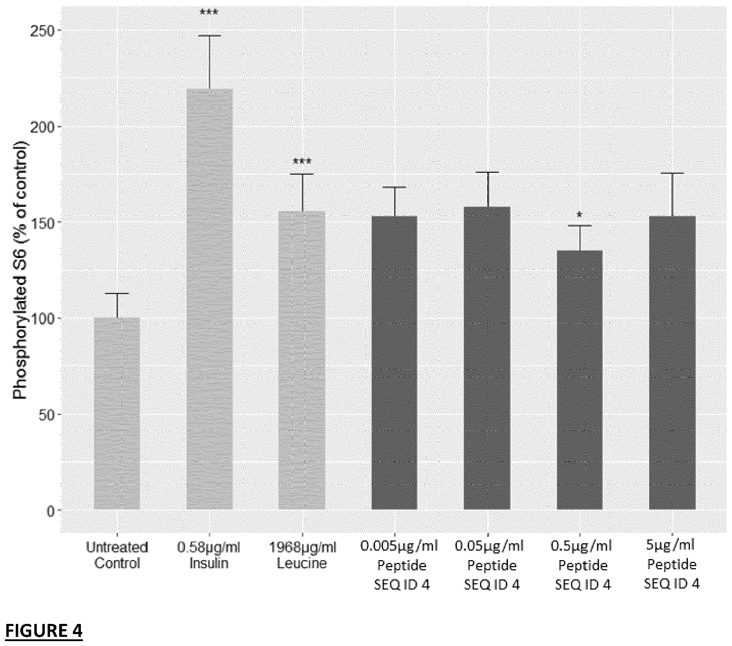

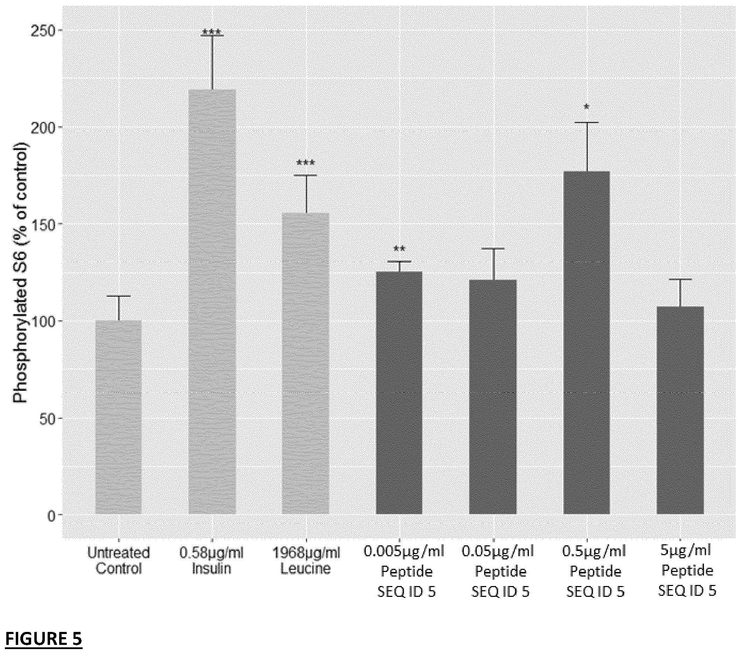

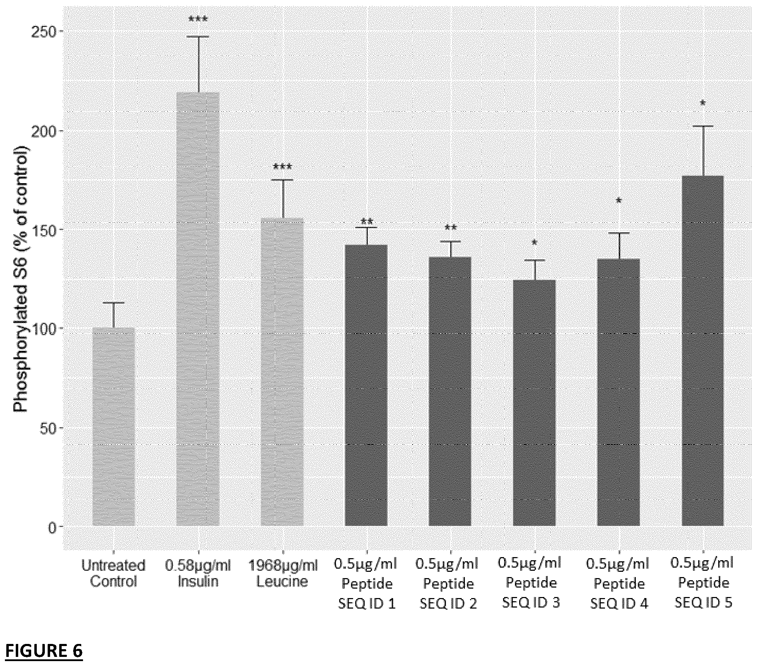

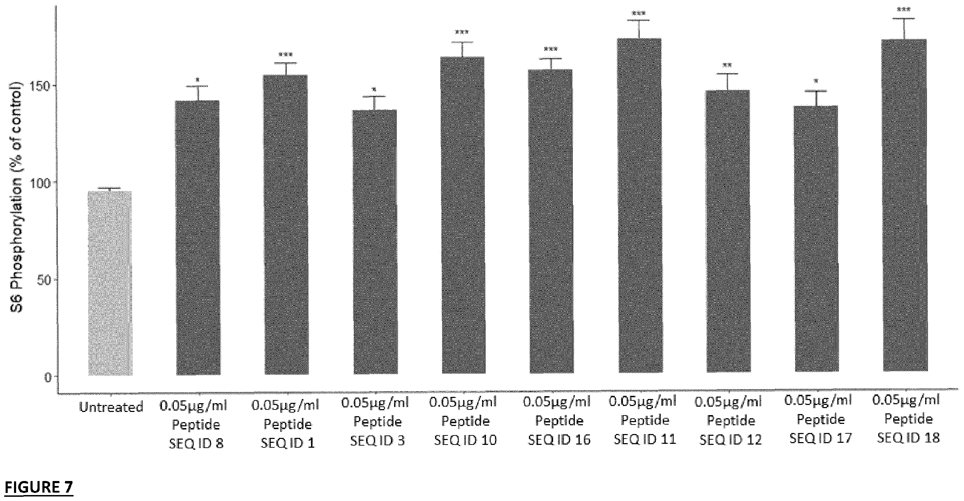

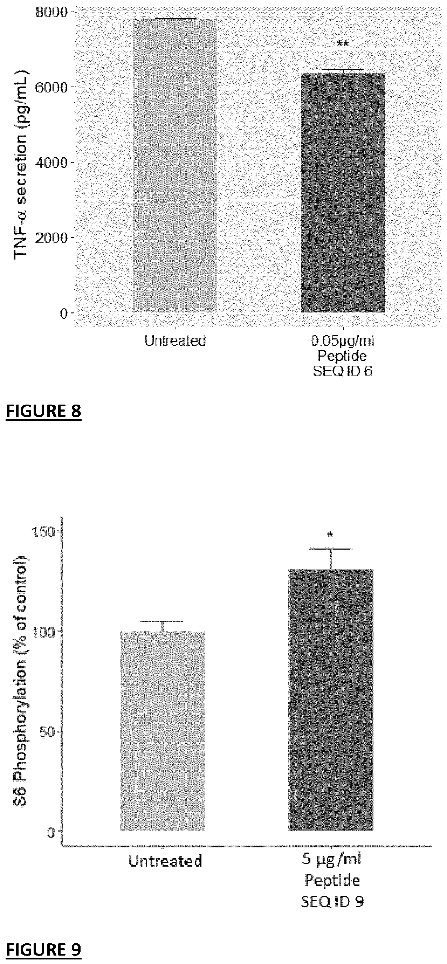

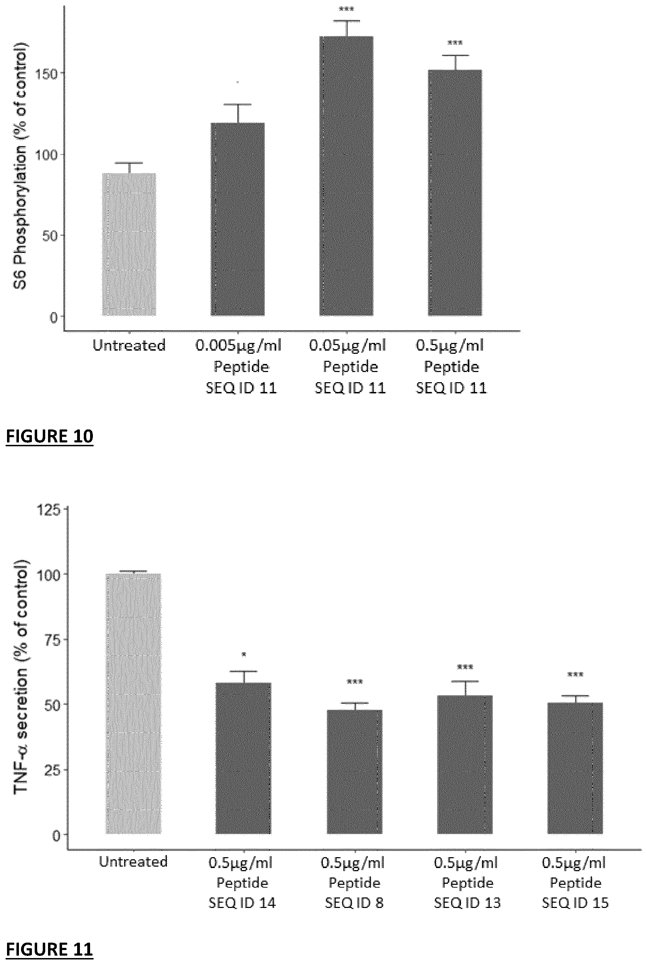

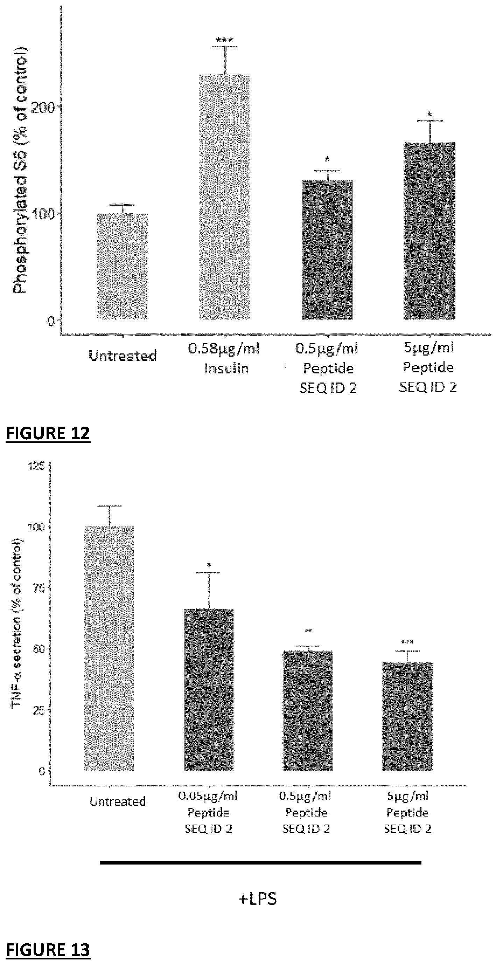

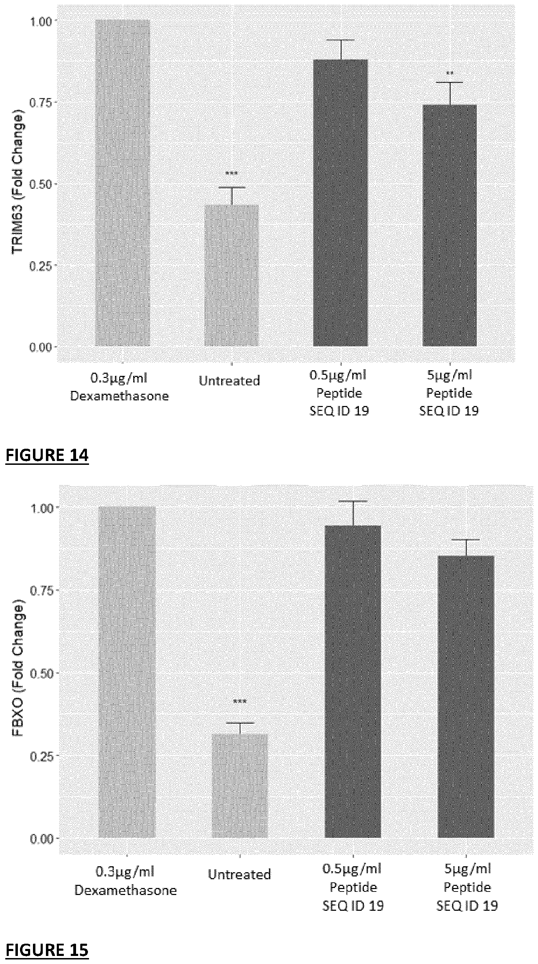

The Applicant has discovered a number of peptides derived from Vicia Faba proteins that are capable of phosphorylating ribosomal protein S6 (rpS6) across a range of concentrations in in-vitro cell assays (Figs. 1-10, 12) in a dose-dependent manner. RPS6 is a key substrate for protein kinases, and is phosphorylated by growth factors and mitogens during in cell growth and cell division. This is a key step in the synthesis of new proteins in skeletal muscle tissue. The peptides described also have the ability to reduce the expression of mRNA Transcripts (TRIM63 and FBXO32) that are directly linked to increase protein degradation, resulting in progressive Skeletal Muscle Atrophy (Figs. 14-15). In addition, increases in muscular atrophy are linked with systemic rise in circulating TNFα.

Some of the peptides described herein also lead to the reduced expression of TNFα in circulating immune cells. The peptides may be used to promote muscle growth and muscle health, reduce muscle loss, and support immune and/or inflammatory response, in subjects, in particular in subjects that exhibit muscle atrophy, for example elderly subjects, physically inactive people, and subjects that have indications characterised by muscle atrophy (i.e. MS and Polio).

According to a first aspect of the present invention, there is provided a peptide comprising or consisting essentially of an amino acid sequence selected from the group consisting of:

| | |
|---|---|
| TIKLPAGT | SEQUENCE ID 1 |
| IEDPGQFPT | SEQUENCE ID 2 |
| HLPSYSPSPQ | SEQUENCE ID 3 |
| KGDIIAIPSGIPY | SEQUENCE ID 4 |
| LDWYKGPT | SEQUENCE ID 5 |
| SRGPIYSN | SEQUENCE ID 6 |
| LERGDTIKIPAGT | SEQUENCE ID 7 |
| TIKIPAGT | SEQUENCE ID 8 |
| SYSPSPQ | SEQUENCE ID 9 |
| IGSSSSPDIYNPQAGRIKT | SEQUENCE ID 10 |
| IDPNGLHLPSYSPSPQL | SEQUENCE ID 11 |
| LVNRDDEEDLRVLDLVIP | SEQUENCE ID 12 |
| ITGQVLHPNGGTVVNA | SEQUENCE ID 13 |
| ALEPDNR | SEQUENCE ID 14 |
| LREQSQQNECQLER | SEQUENCE ID 15 |
| VAGKGIPWDKQDPGEEAIES | SEQUENCE ID 16 |
| VGRRGGQHQQEEESEEQKD | SEQUENCE ID 17 |
| YDEEKEQGEEEIRK | SEQUENCE ID 18 |
| HLPSYSPSP | SEQUENCE ID 19 |
| SYSPSP | SEQUENCE ID 20 |

or a functional variant thereof (hereafter "peptide of the invention").

In one embodiment, the peptide comprises SEQ ID 20. Examples of such peptides include SEQ ID 3, SEQ ID 9, SEQ ID 11 and SEQ ID 19.

In another aspect, the invention provides a modified peptide of the invention. The peptides may be modified by any method described herein, for example by N-terminus, C-terminus, or amino acid side chain modification, by PEGylation, by cyclisation, or by lipidation.

In another aspect, the invention provides a conjugate comprising a peptide of the invention conjugated (generally covalently conjugated) to a binding partner.

The term "peptide of the invention" includes modified peptides and conjugates.

In another aspect, the invention provides a composition comprising one or more or all of the peptides of the invention, for 1, 2, 3, 4, or 5 of the peptides of the invention.

In one embodiment, the composition comprises a peptide comprising SEQ ID 20, for example one, two, three of all of SEQ ID 3, SEQ ID 9, SEQ ID 11 and SEQ ID 19.

In one embodiment, the composition comprises a peptide comprising or consisting essentially of SEQ ID 1 or SEQ ID 8, for example SEQ ID 7 and SEQ ID 8.

In one embodiment, the composition is a powder that optionally comprises additional peptides. The powder may be a protein hydrolysate, which may be supplemented with peptides, and/or may be provided as a comestible powder. In one embodiment, the powder comprises about 0.0001 to about 1.0%, about 0.0001 to about 0.2%, about 0.001 to about 0.1 %, or about 0.001 to about 0.1 %, of the one or more peptides of the invention (w/w). In one embodiment, the powder is substantially free of full proteins. In one embodiment, the composition is a food, beverage or dietary supplement. In another embodiment, the composition is a topical composition.

In one embodiment, the powder comprises the peptides of SEQ ID 1 and 3, typically in amounts of about 0.01 to 0.2% (w/w).

In one embodiment, the powder comprises the peptides of SEQ ID 1, 2, 3 and 5, typically in amounts of about 0.001 to 0.2% (w/w).

In one embodiment, the powder comprises the peptides of SEQ ID 1, 2, 3, 4 and 5, typically in amounts of about 0.001 to 0.2% (w/w).

In another aspect, the invention provides a pharmaceutical composition comprising a therapeutically effective amount of a peptide of the invention in combination with a pharmaceutically acceptable excipient.

In another aspect, the invention provides a method of treating or preventing muscle atrophy in a subject comprising a step of administering a therapeutically effective amount of a peptide or composition of the invention to the subject. The treatment may be administered by means of a pharmaceutical composition, by means of a dietary supplement, or by means of a food or beverage that comprises a peptide or composition of the invention. In one embodiment, the subject is an elderly subject or a subject who is physically inactive, for example a subject with a physical injury.

In another aspect, the invention provides a method of treating or preventing muscle atrophy in a subject with a disease or condition characterised by muscle atrophy, comprising a step of administering a therapeutically effective amount of a peptide or composition of the invention to the subject. Examples of diseases or conditions characterised by muscle atrophy include physical injuries, eating disorders, metabolic disease (including Type I and II diabetes), and diseases characterised by neuronal, muscle or joint degeneration such as ALS, MS, muscular dystrophy, Guillane-Barre syndrome, osteoarthritis, polio, rheumatoid arthritis, spinal muscular atrophy, cachexia, sarcopaenia, malnutrition and polymyositis.

In another aspect, the invention provides a method of promoting muscle synthesis in a subject comprising a step of administering a therapeutically effective amount of a peptide or composition of the invention to the subject.

In another aspect, the invention provides a method of reducing muscle loss in a subject comprising a step of administering a therapeutically effective amount of a peptide or composition of the invention to the subject.

In another aspect, the invention provides a method of supporting or enhancing an immune or inflammatory response in a subject comprising a step of administering a therapeutically effective amount of a peptide or composition of the invention to the subject.

In another aspect, the invention provides a method of protecting muscle during a period of muscle breakdown in a subject (for example during weight exercises) comprising a step of administering a therapeutically effective amount of a peptide or composition of the invention to the subject.

In another aspect, the invention provides a method of increasing the abundance of muscle fibres in a subject (especially Type I or Type II muscle fibres) comprising a step of administering a therapeutically effective amount of a peptide or composition of the invention to the subject.

In any embodiment, the subject may be healthy, young or elderly.

In any embodiment, the peptide or composition may be administered orally (for example in a beverage, food, or pharmaceutical composition).

In any embodiment, the peptide comprises SEQ ID 20. Examples of such peptides include SEQ ID 3, SEQ ID 9, SEQ ID 11 and SEQ ID 19.

In any embodiment, the peptide comprises or consists essentially of SEQ ID 1 or SEQ ID 8, for example SEQ ID 7 and SEQ ID 8.

In another aspect, the invention provides a nucleic acid encoding a peptide of the invention.

In another aspect, the invention provides an expression vector comprising DNA encoding a peptide of the invention, in which the vector is configured for heterologous expression of the peptide of the invention, in a host cell (hereafter "expression vector of the invention").

In another aspect, the invention provides a host cell, especially a bacterium or mammalian producer cell, engineered to heterologously express a peptide of the invention (hereafter "transformed cell of the invention"). In one embodiment, the transformed host cell comprises an expression vector on the invention.

The invention also provides a method of producing a peptide of the invention of the invention, comprising the steps of providing a transformed cell of the invention, culturing the transformed host cell to effect heterologous expression of recombinant peptide of the invention by the host cell, and recovering the recombinant peptide of the invention.

The invention also provides a method of engineering a cell for heterologous expression of a peptide of the invention, comprising the steps of transforming the cell with an expression vector of the invention, whereby the transformed cell is capable of heterologous expression of the peptide of the invention,

Other aspects and preferred embodiments of the invention are defined and described in the other claims set out below.

### Brief Description of the Figures

**Figure. 1****:** rpS6 phosphorylation activity of **SEQ ID 1** at various peptide concentrations.
**Figure. 2****:** rpS6 phosphorylation activity of **SEQ ID 2** at various peptide concentrations.
**Figure. 3****:** rpS6 phosphorylation activity of **SEQ ID 3** at various peptide concentrations.
**Figure. 4****:** rpS6 phosphorylation activity of **SEQ ID 4** at various peptide concentrations.
**Figure. 5****:** rpS6 phosphorylation activity of **SEQ ID 5** at various peptide concentrations.
**Figure. 6****:** rpS6 phosphorylation activity of peptides **SEQ ID 1 to 5** at 0.5 µg/ml.
**Figure. 7****:** rpS6 phosphorylation activity of peptides at 0.05 µg/ml for 20 minutes following a starvation protocol **SEQ ID 8; SEQ ID 1; SEQ ID 3; SEQ ID 10; SEQ ID 16; SEQ ID 11; SEQ ID 12; SEQ ID 17; SEQ ID 18** (One-way ANOVA analysis ;*p<0.05 **p<0.01 ***p<0.001; mean ± SEM; N=4):
**Figure 8****:** Effect of **SEQ ID 6** on S6 phosphorylation. C2C12 cells were treated with peptide (0.005 - 0.5 µg/ml) for 30 mins following a starvation protocol (Student's t test or One-way ANOVA analysis;*p<0.05 **p<0.01 ***p<0.001; mean ± SEM; N=4).
**Figure 9****:** Effect of **SEQ ID 9** which contains motif SYSPSP (SEQ ID 20) on S6 phosphorylation. C2C12 cells were treated with peptide (5 µg/ml) for 30 mins following a starvation protocol (Student's t test or One-way ANOVA analysis;*p<0.05 **p<0.01 ***p<0.001; mean ± SEM; N=4).
**Figure 10****:** Effect of **SEQ ID 11** which contains motif SYSPSP (SEQ ID 20) on S6 phosphorylation. C2C12 cells were treated with peptide (0.005 - 0.5 µg/ml)) for 30 mins following a starvation protocol (Student's t test or One-way ANOVA analysis;*p<0.05 **p<0.01 ***p<0.001; mean ± SEM; N=4).
**Figure 11****:** The effect of peptide treatment on TNF-α secretion. THP-1 macrophages were treated with Nuritas Peptides (0.5µg/ml) for 24 hours and then stimulated with LPS (100ng/ml) for 24 hours. **SEQ ID 14; SEQ ID 8; SEQ ID 13; SEQ ID 15.** (One-way ANOVA analysis; *p<0.05 **p<0.01 ***p<0.001; mean ± SEM; N=4).
**Figure 12****:** Effect of peptide treatment on S6 phosphorylation. C2C12 cells were treated with **SEQ ID 2** (0.5 - 5 µg/ml) for 30 mins following a starvation protocol. (One-way ANOVA analysis;*p<0.05 **p<0.01 ***p<0.001; mean ± SEM; N=3).
**Figure 13****:** The effect of peptide treatment on TNF-α secretion. THP-1 macrophages were treated with **SEQ ID 2** (0.05 - 5 µg/ml) for 24 hours and then stimulated with LPS (100ng/ml) for 24 hours. (One-way ANOVA analysis; *p<0.05 **p<0.01 ***p<0.001; mean ± SEM; N=3).
**Figure 14****:** Effect of Nuritas Peptide on atrophy related gene expression. PCR analysis was carried out on atrophy induced C2C12 cells showing the effect of **SEQ ID 19** (containing the SYSPSP motif) on TRIM63 (N=6) gene expression. Cells were treated with Dexamethasone (0.3µg/ml) for 24hrs, 30 mins prior to the end of Dexamethasone treatment, pep_MU12PE (0.5 - 5µg/ml) was added. (One-way ANOVA analysis; *p<0.05 **p<0.01 ***p<0.001; mean ± SEM).
**Figure 15****:** Effect of Nuritas Peptide on atrophy related gene expression. PCR analysis was carried out on atrophy induced C2C12 cells showing the effect of **SEQ ID** 19 (containing the SYSPSP motif) on FBXO (N=5) gene expression. Cells were treated with Dexamethasone (0.3µg/ml) for 24hrs, 30 mins prior to the end of Dexamethasone treatment, pep_MU12PE (0.5 - 5µg/ml) was added. (One-way ANOVA analysis; *p<0.05 **p<0.01 ***p<0.001; mean ± SEM).

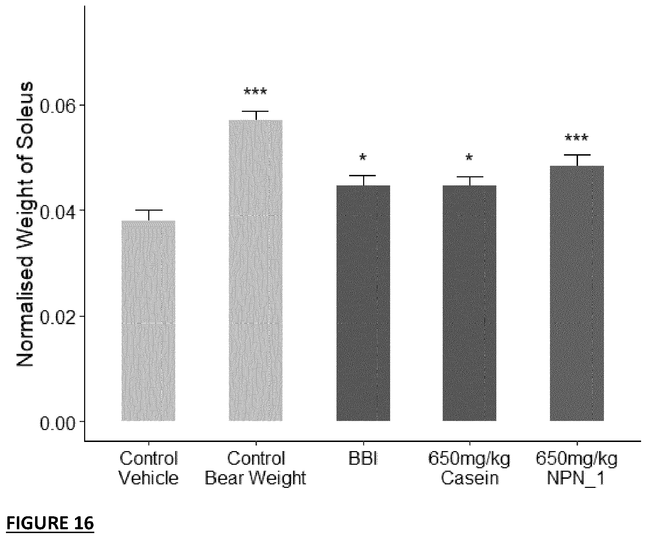

**Figure 16****:** NPN_1 (a powder composition comprising **SEQ ID 3 and 8**) significantly prevented muscle loss in the soleus following unloading. C57BU6 mice were treated with BBI (113.3 mg/kg per day), Casein (650 mg/kg per day) or NPN_1 (650 mg/kg per day) over the course of 18days. (One-way ANOVA analysis; *p<0.05 **p<0.01 ***p<0.001; N=10).

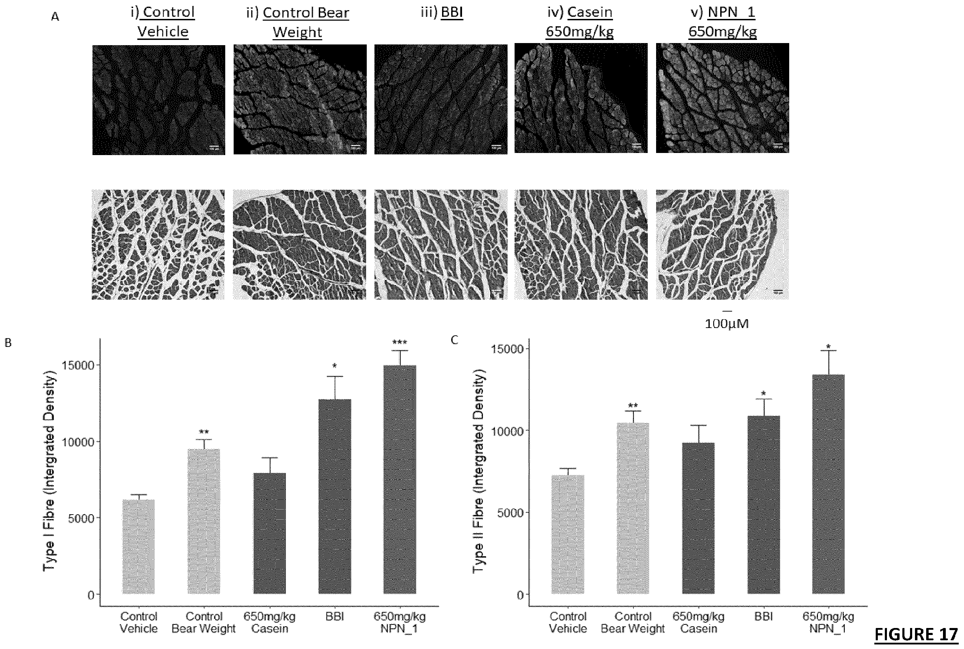

**Figure 17**: NPN_1 reduces the amount of connective tissue and intermuscular fat and increases muscle fibre density. A) Skeletal Muscle Immunostaining on Type-! (Red) & Type Ila (Green) muscle fibre density and hematoxylin and eosin (H&E) staining demonstrating the effect of NPN_1. Quantification of effect of treatment on Type I (B) and Type Ila (C) fibre density (*p<0.05 **p<0.01 ***p<0.001; N=5).

### Detailed Description of the Invention

All publications, patents, patent applications and other references mentioned herein are hereby incorporated by reference in their entireties for all purposes as if each individual publication, patent or patent application were specifically and individually indicated to be incorporated by reference and the content thereof recited in full.

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "disease" is used to define any abnormal condition that impairs physiological function and is associated with specific symptoms. The term is used broadly to encompass any disorder, illness, abnormality, pathology, sickness, condition or syndrome in which physiological function is impaired irrespective of the nature of the aetiology (or indeed whether the aetiological basis for the disease is established). It therefore encompasses conditions arising from infection, trauma, injury, surgery, radiological ablation, poisoning or nutritional deficiencies.

As used herein, the term "treatment" or "treating" refers to an intervention (e.g. the administration of an agent to a subject) which cures, ameliorates or lessens the symptoms of a disease or removes (or lessens the impact of) its cause(s). In this case, the term is used synonymously with the term "therapy".

Additionally, the terms "treatment" or "treating" refers to an intervention (e.g. the administration of an agent to a subject) which prevents or delays the onset or progression of a disease or reduces (or eradicates) its incidence within a treated population. In this case, the term treatment is used synonymously with the term "prophylaxis".

As used herein, an *effective amount* or a *therapeutically effective amount* of a peptide of the invention defines an amount that can be administered to a subject without excessive toxicity, irritation, allergic response, or other problem or complication, commensurate with a reasonable benefit/risk ratio, but one that is sufficient to provide the desired effect, e.g. the treatment or prophylaxis manifested by a permanent or temporary improvement in the subject's condition. The amount will vary from subject to subject, depending on the age and general condition of the individual, mode of administration and other factors. Thus, while it is not possible to specify an exact effective amount, those skilled in the art will be able to determine an appropriate "effective" amount in any individual case using routine experimentation and background general knowledge. A therapeutic result in this context includes eradication or lessening of symptoms, reduced pain or discomfort, prolonged survival, improved mobility and other markers of clinical improvement. A therapeutic result need not be a complete cure.

In the context of treatment and effective amounts as defined above, the term *subject* (which is to be read to include "individual", "animal", "patient" or "mammal" where context permits) defines any subject, particularly a mammalian subject, for whom treatment is indicated. Mammalian subjects include, but are not limited to, humans, domestic animals, farm animals, zoo animals, sport animals, pet animals such as dogs, cats, guinea pigs, rabbits, rats, mice, horses, cattle, cows; primates such as apes, monkeys, orangutans, and chimpanzees; canids such as dogs and wolves; felids such as cats, lions, and tigers; equids such as horses, donkeys, and zebras; food animals such as cows, pigs, and sheep; ungulates such as deer and giraffes; and rodents such as mice, rats, hamsters and guinea pigs. In preferred embodiments, the subject is a human.

The term "peptide" used herein refers to a polymer composed of 5 to 50 amino acid monomers typically via peptide bond linkage. Peptides (including fragments and variants thereof) of and for use in the invention may be generated wholly or partly by chemical synthesis or by expression from nucleic acid. For example, the peptides of and for use in the present invention can be readily prepared according to well-established, standard liquid or, preferably, solid-phase peptide synthesis methods known in the art (see, for example, J. M. Stewart and J. D. Young, Solid Phase Peptide Synthesis, 2nd edition, Pierce Chemical Company, Rockford, Illinois (1984), in M. Bodanzsky and A. Bodanzsky, The Practice of Peptide Synthesis, Springer Verlag, New York (1984). When necessary, any of the peptides employed in the invention can be chemically modified to increase their stability. A chemically modified peptide or a peptide analog includes any functional chemical equivalent of the peptide characterized by its increased stability and/or efficacy in vivo or in vitro in respect of the practice of the invention. The term peptide analog also refers to any amino acid derivative of a peptide as described herein. A peptide analog can be produced by procedures that include, but are not limited to, modifications to side chains, incorporation of unnatural amino acids and/or their derivatives during peptide synthesis and the use of cross-linkers and other methods that impose conformational constraint on the peptides or their analogs. Examples of side chain modifications include modification of amino groups, such as by reductive alkylation by reaction with an aldehyde followed by reduction with NaBH4; amidation with methylacetimidate; acetylation with acetic anhydride; carbamylation of amino groups with cyanate; trinitrobenzylation of amino groups with 2, 4, 6, trinitrobenzene sulfonic acid (TNBS); alkylation of amino groups with succinic anhydride and tetrahydrophthalic anhydride; and pyridoxylation of lysine with pyridoxa-5'-phosphate followed by reduction with NABH4. The guanidino group of arginine residues may be modified by the formation of heterocyclic condensation products with reagents such as 2,3-butanedione, phenylglyoxal and glyoxal. The carboxyl group may be modified by carbodiimide activation via o-acylisourea formation followed by subsequent derivatization, for example, to a corresponding amide. Sulfhydryl groups may be modified by methods, such as carboxymethylation with iodoacetic acid or iodoacetamide; performic acid oxidation to cysteic acid; formation of mixed disulphides with other thiol compounds; reaction with maleimide; maleic anhydride or other substituted maleimide; formation of mercurial derivatives using 4-chloromercuribenzoate, 4-chloromercuriphenylsulfonic acid, phenylmercury chloride, 2-chloromercuric-4-nitrophenol and other mercurials; carbamylation with cyanate at alkaline pH. Tryptophan residues may be modified by, for example, oxidation with N-bromosuccinimide or alkylation of the indole ring with 2-hydroxy-5-nitrobenzyl bromide or sulphonyl halides. Tryosine residues may be altered by nitration with tetranitromethane to form a 3-nitrotyrosine derivative. Modification of the imidazole ring of a histidine residue may be accomplished by alkylation with iodoacetic acid derivatives or N-carbethoxylation with diethylpyrocarbonate. Examples of incorporating unnatural amino acids and derivatives during peptide synthesis include, but are not limited to, use of norleucine, 4-amino butyric acid, 4-amino-3-hydroxy-5-phenylpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids. Peptide structure modification includes the generation of retro-inverso peptides comprising the reversed sequence encoded by D-amino acids.

The term "peptide of the invention" collectively refers to peptides comprising or consisting essentially of an amino acid sequence selected from SEQUENCE ID NO's 1 to 20, and therapeutically effective variants thereof. The peptide of the invention may be a recombinant peptide.

The term "therapeutically effective variant" as applied to a reference peptide means peptides having an amino acid sequence that is substantially identical to the reference peptide, and which is therapeutically effective as defined below. Thus, for example, the term should be taken to include variants that are altered in respect of one or more amino acid residues. Preferably such alterations involve the insertion, addition, deletion and/or substitution of 5 or fewer amino acids, more preferably of 4 or fewer, even more preferably of 3 or fewer, most preferably of 1 or 2 amino acids only. Insertion, addition and substitution with natural and modified amino acids is envisaged. The variant may have conservative amino acid changes, wherein the amino acid being introduced is similar structurally, chemically, or functionally to that being substituted. Generally, the variant will have at least 70% amino acid sequence homology, preferably at least 80% sequence homology, more preferably at least 90% sequence homology, and ideally at least 95%, 96%, 97%, 98% or 99% sequence homology with the reference antibacterial fragment. In this specification, the term "sequence identity" should be understand to comprise both sequence identity and similarity, i.e. a variant (or homolog) that shares 70% sequence identity with a reference sequence is one in which any 70% of aligned residues of the variant (or homolog) are identical to or conservative substitutions of the corresponding residues in the reference sequence across the entire length of the sequence. Sequence identity is the amount of characters which match exactly between two different sequences. Hereby, gaps are not counted and the measurement is relational to the shorter of the two sequences. In terms of "sequence homology", the term should be understood to mean that a variant (or homolog) which shares a defined percent similarity or identity with a reference sequence when the percentage of aligned residues of the variant (or homolog) are either identical to, or conservative substitutions of, the corresponding residues in the reference sequence and where the variant (or homolog) shares the same function as the reference sequence.

This alignment and the percent homology or sequence identity can be determined using software programs known in the art, for example, one alignment program is BLAST, using default parameters. Details of these programs can be found at the following Internet address: http://www.ncbi.nlm.nih.gov/blast/Blast.cgi.

"Therapeutically effective" as applied to a peptide of the invention in the context of muscle synthesis means a peptide that is capable of effecting a significant increase in rpS6 phosphorylation compared with a control in the Phospho-S6 cell assay described below. This applies especially to the peptides of SEQ ID 1-3, 8-12 and 16-20.

"Therapeutically effective" as applied to a peptide of the invention in the context of supporting an immune or inflammatory response means a peptide that is capable of reducing TNF-alpha secretion in THP-1 macrophages compared with a control in the TNF-alpha secretion cell assay described below. This applies especially to the peptides of SEQ ID 2, 8 and 13-15.

"Compositions": The invention also relates to a composition comprising one or more of the peptides of the invention. The peptide, or some or all of the peptide, may be modified or provided as a conjugate. The composition may be a food ingredient powder, food beverage, dietary supplement, pharmaceutical composition, or topical composition. In one embodiment the composition is a sports nutrition product, for example a beverage, snack or supplement. In one embodiment the composition is a beverage. In one embodiment the composition is a bakery product. In one embodiment the composition is a dairy product. In one embodiment the composition is a snack product. In one embodiment the composition is a baked extruded food product. In one embodiment the composition is powdered milk. In one embodiment the composition is an infant formula product. In one embodiment the composition is a confectionary product. In one embodiment the composition is a yoghurt. In one embodiment the composition is a yoghurt drink. In one embodiment the composition is an ice cream product. In one embodiment the composition is a frozen food product. In one embodiment the composition is a breakfast cereal. In one embodiment the composition is a bread. In one embodiment the composition is a flavoured milk drink. In one embodiment the composition is a confectionary bar. In one embodiment the composition is a tea or tea product. In one embodiment the composition is a based extruded snack product. In one embodiment the composition is a fried snack product. In one embodiment the composition is a nutritional supplement. In one embodiment the composition is a sports nutritional product. In one embodiment the composition is a baby food product. In one embodiment the composition is a speciality food product for immunocompromised individuals. In one embodiment the composition is a food for geriatric patients. In one embodiment, the composition is an animal feed. In one embodiment, the composition is an animal feed supplement. In one embodiment, the composition is a medical food.

The composition may be a topical or pharmaceutical composition. The peptides of the invention are used in topical or pharmaceutical composition of this invention at therapeutically effective concentrations to achieve the desired effect; in a preferred form with regards to the total weight of the composition, between 0.00000001 % (in weight) and 20% (in weight); preferably between 0.000001% (in weight) and 15% (in weight), more preferably between 0.0001 % (in weight) and 10% (in weight) and even more preferably between 0.0001% (in weight) and 5% (in weight). Ideally, the peptides of the present invention are preferably used from about 0.00001 % w/w to about 0.5% w/w [0.1 to 5000 ppm], and more preferably from 0.00005 w/w to about 0.05 w/w [0.5 to 500 ppm], and most preferably from about 0.0001 w/w to about 0.01 w/w of the composition [1 to 100 ppm]. Ideally, the peptides of the present invention are preferably used from about 0.0001 % w/w to about 0.004% w/w of the composition.

The dosage of compositions of the invention for use in food products and food or nutritional supplements (i.e. comestible compositions) will be broadly in the 0.2-100 g/day range. In one embodiment, the daily dosage is 1-10 g/day, ideally about 3-8 g/day. In one embodiment, the daily dosage is 10-20 g/day. In one embodiment, the daily dosage is 20-30 g/day. In one embodiment, the daily dosage is 30-40 g/day. In one embodiment, the daily dosage is 10-100 g/day. In one embodiment, the daily dosage is about 5 g/day, ideally about 3-8 g/day. In one embodiment, the dosage is 2-1000 mg/day/kg body weight. In one embodiment, the dosage is 10-500 mg/day/kg body weight. In one embodiment, the dosage is 10-100 mg/day/kg body weight. In one embodiment, the dosage is 30-70 mg/day/kg body weight. The dosage of peptides of the invention for food supplements may be 0.00001 mg-0.01mg per day or dose.

The food product may be a Food for Specific Medicinal Purposes (FSMP) which is defined as foods that are specifically formulated, processed and intended for the dietary management of diseases, disorders or medical conditions of individuals who are being treated under medical supervision. These foods are intended for the exclusive or partial feeding of people whose nutritional requirements cannot be met by normal foods. The dose may be 50-500g per day depending on the age and condition of the patient. When administered as a food for special medicinal purpose, or medical food, the daily dosage may be 50-500g per day.

"Topical compositions": The invention also relates to a topical composition comprising a peptide or composition of the invention. It will be appreciated that the topical composition may comprise a plurality of peptides. In one embodiment, the topical composition comprises substantially all the peptides. The topical composition of the invention may be presented in a formulation selected from the group comprising creams, multiple emulsions, anhydrous compositions, aqueous dispersions, oils, milks, balsams, foams, lotions, gels, cream gels, hydro-alcoholic solutions, hydro-glycolic solutions, cosmetic, personal care product, hydrogels, liniments, sera, soaps, dusting powder, paste, semi solid formulations, liniments, serums, shampoo, conditioner, ointments, any rinse off formulation, talc, mousses, powders, sprays, aerosols, solutions, suspensions, emulsions, syrups, elixirs, polysaccharide films, patches, gel patches, bandages, an adhesive system, water-in-oil emulsions, oil-in-water emulsions, and silicone emulsions.

"Pharmaceutical compositions": A further aspect of the invention relates to a pharmaceutical composition comprising a peptide of the invention or a composition of peptides of the invention, admixed with one or more pharmaceutically acceptable diluents, excipients or carriers. Even though the peptides and compositions of the present invention can be administered alone, they will generally be administered in admixture with a pharmaceutical carrier, excipient or diluent, particularly for human therapy. The pharmaceutical compositions may be for human or animal usage in human and veterinary medicine. Examples of such suitable excipients for the various different forms of pharmaceutical compositions described herein may be found in the "Handbook of Pharmaceutical Excipients, 2nd Edition, (1994), Edited by A Wade and PJ Weller. In particular, formulations for topical delivery are described in Topical drug delivery formulations edited by David Osborne and Antonio Aman, Taylor & Francis, the complete contents of which are incorporated herein by reference. Acceptable carriers or diluents for therapeutic use are well known in the pharmaceutical art, and are described, for example, in Remington's Pharmaceutical Sciences, Mack Publishing Co. (A. R. Gennaro edit. 1985). Examples of suitable carriers include lactose, starch, glucose, methyl cellulose, magnesium stearate, mannitol, sorbitol and the like. Examples of suitable diluents include ethanol, glycerol and water. The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s). Examples of suitable binders include starch, gelatin, natural sugars such as glucose, anhydrous lactose, free-flow lactose, betalactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Preservatives, stabilizers, dyes and even flavouring agents may be provided in the pharmaceutical composition. Examples of preservatives include sodium benzoate, sorbic acid and esters of p hydroxybenzoic acid. Antioxidants and suspending agents may be also used.

The peptide or composition of the invention may be adapted for topical, oral, rectal, parenteral, intramuscular, intraperitoneal, intra-arterial, intrabronchial, subcutaneous, intradermal, intravenous, nasal, vaginal, buccal or sublingual routes of administration. For oral administration, particular use is made of compressed tablets, pills, tablets, gellules, drops, and capsules. Preferably, these compositions contain from 1 to 250 mg and more preferably from 10-100 mg, of active ingredient per dose. Other forms of administration comprise solutions or emulsions which may be injected intravenously, intra-arterial, subcutaneously, intradermally, intraperitoneally or intramuscularly, and which are prepared from sterile or sterilisable solutions. The pharmaceutical compositions of the present invention may also be in form of suppositories, vaginal rings, pessaries, suspensions, emulsions, lotions, ointments, creams, gels, sprays, solutions or dusting powders. The composition of the invention may be formulated for topical delivery. Topical delivery generally means delivery to the skin, but can also mean delivery to a body lumen lined with epithelial cells, for example the lungs or airways, the gastrointestinal tract, the buccal cavity. In particular, formulations for topical delivery are described in Topical drug delivery formulations edited by David Osborne and Antonio Aman, Taylor & Francis, the complete contents of which are incorporated herein by reference. Compositions or formulations for delivery to the airways are described in O'Riordan et al (Respir Care, 2002, Nov. 47), EP2050437, WO2005023290, US2010098660, and US20070053845. Composition and formulations for delivering active agents to the iluem, especially the proximal iluem, include microparticles and microencapsulates where the active agent is encapsulated within a protecting matrix formed of polymer or dairy protein that is acid resistant but prone to dissolution in the more alkaline environment of the ileum. Examples of such delivery systems are described in EP1072600.2 and EP13171757.1. An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required. Injectable forms may contain between 10-1000 mg, preferably between 10-250 mg, of active ingredient per dose.

Compositions may be formulated in unit dosage form, i.e., in the form of discrete portions containing a unit dose, or a multiple or sub-unit of a unit dose.

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention. Depending upon the need, the agent may be administered at a dose of from 0.01 to 30 mg/kg body weight, such as from 0.1 to 10 mg/kg, more preferably from 0.1 to 1 mg/kg body weight. In an exemplary embodiment, one or more doses of 10 to 300 mg/day or more preferably, 10 to 150 mg/day, will be administered to the patient for the treatment of an inflammatory disorder.

In a particularly preferred embodiment, the methods and uses of the invention involve administration of a peptide or composition of the invention in combination with one or more other active agents, for example, existing antibacterial drugs or pharmacological enhancers available on the market. In such cases, the compounds of the invention may be administered consecutively, simultaneously or sequentially with the one or more other active agents.

In one embodiment of the invention, the peptide of the invention may be administered in the form of a conjugate comprising the peptide, a linker, and an antibody molecule intended to increase the half-life of the conjugate in-vivo.

### Modified Peptides

"Modified peptides": In one embodiment, the peptides of the invention (including peptide variants) may be a modified peptide. The term "modified peptide" is used interchangeably with the term derivative of the peptide. In one embodiment, the term "modified peptide" means a peptide that is modified to exhibit one or more of the following properties compared with the unmodified peptide: increase plasma half-life; increase the lipophilicity of the peptide; increase the renal clearance of the modified peptide; and increase the resistance of the modified peptide to proteolytic degradation, while typically retaining the rpS6 phosphorylation activity. Various methods of modifying a peptide of the invention to exhibit these properties are disclosed herein, including conjugating the peptide with a binding partner (for example an albumin binding small molecule, large polymer, long life plasma protein, or antibody or antibody-fragment), cyclisation, addition of N- or C-terminal, or side chain, protecting groups, replacing L-amino acids with D-isomers, amino acid modification, increased plasma protein binding, increased albumin binding The modified peptide includes but is not limited to a peptide which has been substituted with one or more groups as defined herein, or conjugated with a binding partner, or cyclized. Generally, the peptide is modified to increase it half-life in-vivo in an animal. Various methods of modification are provided below.

In one embodiment, the modification may be any modification that provides the peptides and or the composition of the invention with an increased ability to penetrate a cell. In one embodiment, the modification may be any modification that increases the half-life of the composition or peptides of the invention. In one embodiment, the modification may be any modification that increases activity of the composition or peptides of the invention. In one embodiment, the modification may be any modification that increases selectivity of the composition or peptides of the invention.

In one embodiment, the group is a protecting group. The protecting group may be an N-terminal protecting group, a C-terminal protecting group or a side-chain protecting group. The peptide may have one or more of these protecting groups.

The person skilled in the art is aware of suitable techniques to react amino acids with these protecting groups. These groups can be added by preparation methods known in the art, for example the methods as outlined in paragraphs [0104] to [0107] of US2014120141. The groups may remain on the peptide or may be removed. The protecting group may be added during synthesis.

In an embodiment of the invention the peptides may be substituted with a group selected from one or more straight chain or branched chain, long or short chain, saturated, or unsaturated, substituted with a hydroxyl, amino, amino acyl, sulfate or sulphide group or unsubstituted having from 1 to 29 carbon atoms. N-acyl derivatives include acyl groups derived from acetic acid, capric acid, lauric acid, myristic acid, octanoic acid, palmitic acid, stearic acid, behenic acid, linoleic acid, linolenic acid, lipoic acid, oleic acid, isosteric acid, elaidoic acid, 2-ethylhexaneic acid, coconut oil fatty acid, tallow fatty acid, hardened tallow fatty acid, palm kernel fatty acid, lanolin fatty acid or similar acids. These may be substituted or unsubstituted. When substituted they are preferably substituted with hydroxyl, or sulphur containing groups such as but not limited to SO3H, SH, or S-S.

In an embodiment of the current invention, the peptide is R1-X- R2.

R1 and/or R2 groups respectively bound to the amino-terminal (N-terminal) and carboxyl-terminal (C-terminal) of the peptide sequence.

In one embodiment, the peptide is R1-X. Alternatively, the peptide is X- R2.

Preferably, R1 is H, C1-4 alkyl, acetyl, benzoyl or trifluoroacetyl;
X is the peptide of the invention;
R2 is OH or NH2.

In an embodiment, R 1 is selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, substituted or unsubstituted aralkyl, Tert-butyloxycarbonyl, 9-fluorenylmethyloxycarbonyl (Fmoc) and R5-CO-, wherein R5 is selected from the group formed by H, a non-cyclic substituted or unsubstituted aliphatic group, substituted or unsubstituted alicyclyl, substituted or unsubstituted aryl, substituted or unsubstituted aralkyl, substituted or unsubstituted heterocyclyl and substituted or unsubstituted heteroarylalkyl;
R2 is selected from the group formed by -NR3R4, -OR3 and -SR3, wherein R3 and R4 are independently selected from the group formed by H, a non-cyclic substituted or unsubstituted aliphatic group, substituted or unsubstituted alicyclyl, substituted or unsubstituted heterocyclyl, substituted or unsubstituted heteroarylalkyl, substituted or unsubstituted aryl, and substituted or unsubstituted aralkyl; and with the condition that R1 and R2 are not α-amino acids.

In accordance with another preferred embodiment, R2 is -NR3R4, -OR 3 or -SR 3 wherein R3 and R4 are independently selected from the group formed by H, substituted or unsubstituted C 1-C 24 alkyl, substituted or unsubstituted C2-C 24 alkenyl, Tert-butyloxycarbonyl, 9-fluorenylmethyloxycarbonyl (Fmoc), substituted or unsubstituted C2-C 24 alkynyl, substituted or unsubstituted C3-C 24 cycloalkyl, substituted or unsubstituted C 5-C 24 cycloalkenyl, substituted or unsubstituted C8-C 24 cycloalkynyl, substituted or unsubstituted C 6-C 30 aryl, substituted or unsubstituted C7-C24 aralkyl, substituted or unsubstituted heterocyclyl ring of 3-10 members, and substituted or unsubstituted heteroarylalkyl of 2 to 24 carbon atoms and 1 to 3 atoms other than carbon wherein the alkyl chain is of 1 to 6 carbon atoms. Optionally, R 3 and R 4 can be bound by a saturated or unsaturated carbon-carbon bond, forming a cycle with the nitrogen atom. More preferably R 2 is -NR3R4 or -OR 3, wherein R3 and R4 are independently selected from the group formed by H, substituted or unsubstituted C1-C 24 alkyl, substituted or unsubstituted C2-C24 alkenyl, substituted or unsubstituted C2-C24 alkynyl, substituted or unsubstituted C3-C10 cycloalkyl, substituted or unsubstituted C6-C 15 aryl and substituted or unsubstituted heterocyclyl of 3-10 members, substituted or unsubstituted heteroarylalkyl with a ring of 3 to 10 members and an alkyl chain of 1 to 6 carbon atoms. More preferably R3 and R4 are selected from the group formed by H, methyl, ethyl, hexyl, dodecyl, or hexadecyl. Even more preferably R3 is H and R4 is selected from the group formed by H, methyl, ethyl, hexyl, dodecyl, or hexadecyl. In accordance with an even more preferred embodiment, R2 is selected from -OH and -NH2.

In accordance with another embodiment of this invention R 1 is selected from the group formed by H, acetyl, lauroyl, myristoyl or palmitoyl, and R2 is -NR3R 4 or -OR3 wherein R3 and R4 are independently selected from H, methyl, ethyl, hexyl, dodecyl and hexadecyl, preferably R2 is -OH or -NH2. More preferably, R1 is acetyl or palmitoyl and R2 is -NH2.

In a preferred embodiment, the acyl group is bound to the N-terminal end of at least one amino acid of the peptide.

In an embodiment of the invention, the peptide is modified to comprise a side chain protecting group. The side chain protecting group may be one or more of the group comprising benzyl or benzyl based groups, t-butyl-based groups, benzyloxy-carbonyl (Z) group, and allyloxycarbonyl (alloc) protecting group. The side chain protecting group may be derived from an achiral amino acid such as achiral glycine. The use of an achiral amino acid helps to stabilise the resultant peptide and also facilitate the facile synthesis route of the present invention. Preferably, the peptide further comprises a modified C-terminus, preferably an amidated C-terminus. The achiral residue may be alpha-aminoisobutyric acid (methylalaine). It will be appreciated that the specific side chain protecting groups used will depend on the sequence of the peptide and the type of N-terminal protecting group used. In one embodiment of the invention the peptide is conjugated, linked or fused to one or more polyethylene glycol polymers or other compounds, such as molecular weight increasing compounds. The molecular weight increasing compound is any compound that will increase the molecular weight, typically by 10% to 90%, or 20% to 50% of the resulting conjugate and may have a molecular weight of between 200 and 20, 000, preferably between 500 and 10, 000. The molecular weight increasing compound may be PEG, any water-soluble(amphiphilic or hydrophilic) polymer moiety, homo or co-polymers of PEG, a monomethyl-subsitututed polymer of PEG (mPEG) and polyoxyethylene glycerol (POG), polyamino acids such as poly-lysine, poly-glutamic acid, poly-aspartic acid, particular those of L conformation, pharmacologically inactive proteins such as albumin, gelatin, a fatty acid, olysaccharide, a lipid amino acid and dextran. The polymer moiety may be straight chained or branched and it may have a molecular weight of 500 to 40000Da, 5000 to 10000 Da, 10000 to 5000, Da. The compound may be any suitable cell penetrating compound, such as tat peptide, penetratin, pep-1. The compound may be an antibody molecule. The compound may be a lipophilic moiety or a polymeric moiety.

The lipophilic substituent and polymeric substituents are known in the art. The lipophilic substituent includes an acyl group, a sulphonyl group, an N atom, an O atom or an S atom which forms part of the ester, sulphonyl ester, thioester, amide or sulphonamide. The lipophilic moiety may include a hydrocarbon chain having 4 to 30 C atoms, preferably between 8 and 12 C atoms. It may be linear or branched, saturated or unsaturated. The hydrocarbon chain may be further substituted. It may be cycloalkane or heterocycloalkane. The peptide may be modified at the N-terminal, C-terminal or both. The polymer or compound is preferably linked to an amino, carboxyl or thio group and may be linked by N-termini or C-termini of side chains of any amino acid residue. The polymer or compound may be conjugated to the side chain of any suitable residue.

The polymer or compound may be conjugated via a spacer. The spacer may be a natural or unnatural amino acid, succinic acid, lysyl, glutamyl, asparagyl, glycyl, beta-alanyl, gamma-amino butanoyl.

The polymer or compound may be conjugated via an ester, a sulphonyl ester, a thioester, an amide, a carbamate, a urea, a sulphonamide.

A person skilled in the art is aware of suitable means to prepare the described conjugate.

Peptides can be chemically modified by covalent conjugation to a polymer to increase their circulating half-life, for example. Exemplary polymers and methods to attach such polymers to peptides are illustrated in, e.g., U.S. Pat. Nos. 4,766,106; 4,179,337; 4,495,285; and 4,609,546. Additional illustrative polymers include polyoxyethylated polyols and polyethylene glycol (PEG) moieties.

The peptides of the invention may be subjected to one or more modifications for manipulating storage stability, pharmacokinetics, and/or any aspect of the bioactivity of the peptide, such as, e.g., potency, selectivity, and drug interaction. Chemical modification to which the peptides may be subjected includes, without limitation, the conjugation to a peptide of one or more of polyethylene glycol (PEG), monomethoxy-polyethylene glycol, dextran, poly-(N-vinyl pyrrolidone) polyethylene glycol, propylene glycol homopolymers, a polypropylene oxide/ethylene oxide co-polymer, polypropylene glycol, polyoxyethylated polyols (e.g., glycerol) and polyvinyl alcohol, colominic acids or other carbohydrate based polymers, polymers of amino acids, and biotin derivatives. PEG conjugation of proteins at Cys residues is disclosed, e.g., in Goodson, R. J. & Katre, N. V. (1990) Bio/Technology 8, 343 and Kogan, T. P. (1992) Synthetic Comm. 22, 2417.

Modified peptides also can include sequences in which one or more residues are modified (i.e., by phosphorylation, sulfation, acylation, PEGylation, etc.), and mutants comprising one or more modified residues with respect to a parent sequence. Amino acid sequences may also be modified with a label capable of providing a detectable signal, either directly or indirectly, including, but not limited to, radioisotope, fluorescent, and enzyme labels. Fluorescent labels include, for example, Cy3, Cy5, Alexa, BODIPY, fluorescein (e.g., FluorX, DTAF, and FITC), rhodamine (e.g., TRITC), auramine, Texas Red, AMCA blue, and Lucifer Yellow. Preferred isotope labels include 3H, 14C, 32 P, 35S, 36Cl, 51Cr, 57Co, 58Co, 59Fe, 90Y, 125I, 131I, and 286Re. Preferred enzyme labels include peroxidase, β-glucuronidase, β-D-glucosidase, β-D-galactosidase, urease, glucose oxidase plus peroxidase, and alkaline phosphatase (see, e.g., U.S. Pat. Nos. 3,654,090; 3,850,752 and 4,016,043). Enzymes can be conjugated by reaction with bridging molecules such as carbodiimides, diisocyanates, glutaraldehyde, and the like. Enzyme labels can be detected visually, or measured by calorimetric, spectrophotometric, fluorospectrophotometric, amperometric, or gasometric techniques. Other labeling systems, such as avidin/biotin, Tyramide Signal Amplification (TSA^{™}), are known in the art, and are commercially available (see, e.g., ABC kit, Vector Laboratories, Inc., Burlingame, Calif.; NEN^{®} Life Science Products, Inc., Boston, Mass.).

In an embodiment, the peptide, variant and/or composition is modified to increase drug performance ability. In an embodiment, the peptide, variant and/or composition is modified to increase stability, permeability, maintain potency, avoid toxicity and/or to increase half-life. The modification may be as described above. For example, the modification may be to protect the N and C-terminus, it may be a modified amino acid, cyclisation, replacement of an amino acid, and/or conjugation to macromolecules or large polymers or long life plasma proteins. Strategies to extend a half-life may be as described by Strohl, et al (BioDrugs, 2015), Schlapschy, et al (Protein Eng Des Sel. 2013), Podust, VN, et al (Protein Eng Des Sel. 2013), Zhang, Let al (Curr Med Chem. 2012), Gaberc-Porekar, V, et al (Curr Opin Drug Discov Devel. 2008). Examples include using PEGylation, lipidation (covalent binding of fatty acids to peptide side chains), fusion to Fc domains and human serum albumin, fusion with a hydrophilic amino acid polymer, e.g. XTEN or PAS, and/or fusion with half-life extension proteins.

Modification of peptides to extend the in-vivo half-life of the peptide is described in the literature, for example:
Strategies to improve plasma half life time of peptide and protein drugs. Werle M, Bernkop-Schnürch A. Amino Acids. 2006 Jun;30(4):351-67.

Due to the obvious advantages of long-acting peptide and protein drugs, strategies to prolong plasma half life time of such compounds are highly on demand. Short plasma half life times are commonly due to fast renal clearance as well as to enzymatic degradation occurring during systemic circulation. Modifications of the peptide/protein can lead to prolonged plasma half life times. By shortening the overall amino acid amount of somatostatin and replacing L: -analogue amino acids with D: -amino acids, plasma half life time of the derivate octreotide was 1.5 hours in comparison to only few minutes of somatostatin. A PEG(2,40 K) conjugate of INF-alpha-2b exhibited a 330-fold prolonged plasma half life time compared to the native protein. It was the aim of this review to provide an overview of possible strategies to prolong plasma half life time such as modification of N- and C-terminus or PEGylation as well as methods to evaluate the effectiveness of drug modifications. Furthermore, fundamental data about most important proteolytic enzymes of human blood, liver and kidney as well as their cleavage specificity and inhibitors for them are provided in order to predict enzymatic cleavage of peptide and protein drugs during systemic circulation.

Strategic Approaches to Optimizing Peptide ADME Properties. Li Di AAPS J. 2015 Jan; 17(1): 134-143.

### Strategies to Stabilize Peptides from Proteolysis

Many approaches are available to enhance stability of peptides through structure modification. Some approaches not only improve stability, but also enhance other ADME properties, e.g., cyclization can increase stability and permeability; conjugation to macromolecules can improve stability and reduce renal clearance. It is important to maintain potency and avoid toxicity while improving stability and ADME properties of peptides.

### • Protecting N- and C-terminus

A number of proteolytic enzymes in blood/plasma, liver or kidney are exopeptidases, aminopeptidases and carboxypeptidases and they break down peptide sequences from the N- and C-termini. Modification of the N- or/and C-termini can often improve peptide stability. Many examples have reported that N-acetylation, and C-amidation increase resistance to proteolysis.

### • Replacing L-amino acids with D-amino acids

Substituting natural L-amino acids with nonnatural D-amino acids decreases the substrate recognition and binding affinity of proteolytic enzymes and increases stability. One example is vasopressin, which contains an L-Arg and has a half-life of 10-35 min in humans. The D-Arg analog, desmopressin, has a half-life of 3.7 h in healthy human volunteers. In the study of a bicyclic peptide inhibitor of the cancer-related protease urokinase-type plasminogen activator (uPA), replacement of a specific glycine with a D-serine not only improves potency by 1.8-fold but also increases stability by 4-fold in mouse plasma.

### • Modification of amino acids

Modification of natural amino acids can improve the stability of peptides by introducing steric hindrance or disrupting enzyme recognition . For example, gonadotropin-releasing hormone has a very short half-life (minutes), while buserelin, in which one Gly is replaced with a t-butyl-D-Ser and another Gly is substituted by ethylamide, has a much longer half-life in humans.

### • Cyclization

Cyclization introduces conformation constraint, reduces the flexibility of peptides, and increases stability and permeability. Depending on the functional groups, peptides can be cyclized head-to-tail, head/tail-to-side-chain, or side-chain-to-side-chain. Cyclization is commonly accomplished through lactamization, lactonization, and sulfide-based bridges. Disulfide bridges create folding and conformational constraints that can improve potency, selectivity, and stability. A number of disulfide bond-rich peptides are on the market or in preclinical or clinical development, e.g., linaclotide, lepirudin, and ziconotide. A number of method of cyclization of peptides is described in Davies et al. (J. Peptide Sci, 9: 471-501 (2003)), including on-resin cyclisation, side-chain cyclisation (lactam bridges), cyclisation via orthogonal coupling, enzyme catalysed cyclisation, cyclisation specific to the size of the peptide, and cyclic peptides containing thiazole/oxazole rings.

### • Conjugation to Macromolecules

Conjugation to macromolecules (e.g., polyethylene glycol (PEG), albumin) is an effective strategy to improve stability of peptides and reduce renal clearance.

### Renal Clearance

Many peptides exhibit promising in vitro pharmacological activity but fail to demonstrate in vivo efficacy due to very short in vivo half-life (minutes). The rapid clearance and short half-life of peptides hamper their development into successful drugs. The main causes of rapid clearance of peptides from systemic circulation are enzymatic proteolysis or/and renal clearance. The glomeruli have a pore size of ~8 nm, and hydrophilic peptides with MW <2-25 kDa are susceptible to rapid filtration through the glomeruli of the kidney. Since peptides are not easily reabsorbed through the renal tubule, they frequently have high renal clearance and short half-life. Other minor routes of peptide clearance are endocytosis and degradation by proteasome and the liver. Comparison between systemic and renal clearance in animal models provides useful information on whether renal clearance is likely to be a major elimination pathway.

For renal-impaired patients, dose adjustment may be needed for peptide drugs to avoid accumulation and higher drug exposure, as inappropriate dosing in patients with renal dysfunction can cause toxicity or ineffective therapy. Several strategies have been developed to reduce peptide renal clearance and prolong half-life. These will be reviewed next.

### • Increase plasma protein binding

Renal clearance of peptides is reduced when they are bound to membrane proteins or serum proteins. An example is the cyclic peptide drug octreotide, a treatment for endocrine tumors, which has about 100 min half-life in humans due to binding to lipoproteins (fraction unbound 0.65)

### • Covalent Linkage to Albumin-Binding Small Molecules

Covalently attaching albumin-binding small molecules to peptides can reduce glomerular filtration, improve proteolytic stability, and prolong half-life by indirectly interacting with albumin through the highly bound small molecules.

### • Conjugation to Large Polymers

Conjugation of peptides to large synthetic or natural polymers or carbohydrates can increase their molecular weight and hydrodynamic volume, thus reducing their renal clearance. The common polymers used for peptide conjugation are PEG, polysialic acid (PSA), and hydroxyethyl starch (HES).

### • Fusion to Long-Live Plasma Proteins

Plasma proteins, such as albumin and immunoglobulin (IgG) fragments, have long half-lives of 19-21 days in humans. Because of the high MW (67-150 kDa), these proteins have low renal clearance, and their binding to neonatal Fc receptor (FcRn) reduces the elimination through pinocytosis by the vascular epithelium. Covalent linkage of peptides to albumin or IgG fragments can reduce renal clearance and prolong half-life.

Fusion Proteins for Half-Life Extension of Biologics as a Strategy to Make Biobetters William R. Strohl BioDrugs. 2015; 29(4): 215-239.

Schlapschy, M, Binder, U, Borger, C et al. PASYlation: a biological alternative to PEGylation for extending the plasma half-life of pharmaceutically active proteins. Protein Eng Des Sel. 2013;26(8):489-501.

Podust, VN, Sim, BC, Kothari, D et al. Extension of in vivo half-life of biologically active peptides via chemical conjugation to XTEN protein polymer. Protein Eng Des Sel. 2013;26(11):743-53.

Zhang, L, Bulaj, G. Converting Peptides into Drug Leads by Lipidation. Curr Med Chem. 2012;19(11):1602-18.

Gaberc-Porekar, V, Zore, I, Podobnik, B et al. Obstacles and pitfalls in the PEGylation of therapeutic proteins. Curr Opin Drug Discov Devel. 2008;11(2):242-50.

By Dr Ronald V. Swanson - Long live peptides evolution of peptide half-life extension technologies and emerging hybrid approaches. From Drug Discovery World on line. Spring 2014

### PEGylation

The attachment of long chains of the hydrophilic polymer polyethylene glycol to molecules of interest, PEGylation was originally conceived as a modification to prevent the recognition of foreign proteins by the immune system and, thereby, enable their utility as therapeutics. Once formed, antibodies against unmodified drugs can rapidly neutralise and clear protein drugs. Unexpectedly, PEGylation improved the pharmacokinetics of the proteins even in the absence of anti-drug antibodies1. Simply by making drug molecules larger, PEGylation led to the drug being filtered more slowly by the kidneys. The empirical observation that increasing size or hydrodynamic radius led to reduced renal clearance and increased half-life then became the dominant rationale for the PEGylation of protein and peptide drugs. PEGylation can have a variety of effects on the molecule including making proteins or peptides more water-soluble and protecting them from degradation by proteolytic enzymes. PEGylation can also impact the binding of therapeutic proteins to their cognate cellular receptors, usually reducing the affinity. Changes in the size, structure and attachment mode of PEG polymers can affect the biological activity of the attached drug.

The first-generation PEGylation methods were filled with challenges. However, the chemistry of PEGylation is quite simple. The process involves the covalent attachment of polyethylene glycol chains to reactive side chains of a protein or peptide. For example, PEG is easily attached to the -amino groups of lysine on the surface of proteins or peptides2. The reaction is pH-dependent. At high pH (8.0 or higher), lysine side chain amino groups are covalently attached to PEG through N-hydroxy succinimides. This method typically results in a family of products containing different numbers of PEG chains attached at different sites on a protein rather than a single discrete products. The first approved PEGylated pharmaceuticals were Pegademase bovine (PEGylated bovine adenosine deamidase) as enzyme replacement therapy for severe combined immunodeficiency and Pegaspargase (PEGylated asparaginase) for treatment of acute lymphoblastic leukaemia1. These drugs were complex mixtures of various PEGylated species, but with improved properties for therapy over native enzymes, including increased serum half-life and decreased immunogenicity of the proteins. Due to the inherent polydispersity of the PEG, quality and batch-to-batch reproducibility was difficult. Despite this limitation, two PEGylated interferons, (Peginterferon alfa-2b and Peginterferon alfa-2a) that are heterogeneous populations of numerous mono-PEGylated positional isomers, have been FDA-approved for the treatment of hepatitis C. These drugs were brought to market in 2001 and 2002, respectively.

A variety of enhancements and variations have been made to the fundamental PEGylation technology. Second-generation PEGylation processes introduced the use of branched structures as well as alternative chemistries for PEG attachment. In particular, PEGs with cysteine reactive groups such as maleimide or iodoacetamide allow the targeting of the PEGylation to a single residue within a peptide or protein reducing the heterogeneity of the final product but not eliminating it due to the polydispersity of the PEG itself.

While the original rationale for PEGylation was to reduce immunogenicity; nevertheless, there have been a few examples of immunogenic PEGylated proteins. One example is PEGylated urate oxidase, an enzyme that lowers the plasma urate level in patients with gout. In clinical trials, a relatively high percentage of patients with gout did not respond to the therapy and developed antibodies that were specific for PEG, but not for the uricase protein2. PEGylated liposomes, also generally thought to be non-immunogenic, have been found to be immunogenic in some studies. PEGylated liposomes elicit a strong anti-PEG immunoglobulin M (IgM) response. In addition, multiple injections of PEG-glucuronidase were shown to elicit the generation of specific anti-PEG IgM antibodies, thus accelerating the clearance of PEG-modified proteins from the body.

A major potential drawback of using PEG as a modifier is that it is non-biodegradable. The US Food and Drug Administration (FDA) has approved PEG for use as a vehicle in pharmaceuticals, including injectable, topical, rectal and nasal formulations. PEG shows little toxicity and is eliminated from the body intact by either the kidneys (for PEGs < 30 kDa) or in the feces (for PEGs >20 kDa)1. Repeated administration of some PEGylated proteins to animals has resulted in observations of renal tubular cellular vacuolation. Recently, vacuolation of choroid plexus epithelial cells has also been seen in toxicity studies with proteins conjugated with large (≥40 kDa) PEGs. The choroid plexus epithelial cells produce cerebrospinal fluid and form the blood CSF barrier. The long-term negative consequences of cellular vacuolation are unclear, but it does represent an undesirable consequence for some potential therapeutics. One possible alternative would be substitution of a biodegradable polymer in place of PEG. Polymers, such as hydroxyethyl starch (HES) are a possible alternative. HES is non-toxic and biodegradable and used as a blood expander. A process of HESylation would function similarly to PEGylation in reducing renal clearance through increasing a peptide's hydrodynamic radius but may confer a lower propensity for accumulation due to biodegradability. However, HES and other proposed biodegradable polymer PEG alternatives are, like PEG, polydisperse making characterisation of the final product and metabolites difficult. One emerging solution which mitigates both concerns is to use defined polypeptides as the polymer component; this approach will be discussed later in the article.

### Lipidation

A second major chemical modification method to increase peptide half-life is lipidation which involves the covalent binding of fatty acids to peptide side chains4. Originally conceived of and developed as a method for extending the half-life of insulin, lipidation shares the same basic mechanism of half-life extension as PEGylation, namely increasing the hydrodynamic radius to reduce renal filtration. However, the lipid moiety is itself relatively small and the effect is mediated indirectly through the non-covalent binding of the lipid moiety to circulating albumin. A large (67 KDa) and highly abundant protein in human serum (35- 50g/L), albumin naturally functions to transport molecules, including lipids, throughout the body. Binding to plasma proteins can also protect the peptide from attacks by peptidases through steric hindrance, again akin to what is seen with PEGylation. One consequence of lipidation is that it reduces the water-solubility of the peptide but engineering of the linker between the peptide and the fatty acid can modulate this, for example by the use of glutamate or mini PEGs within the linker. Linker engineering and variation of the lipid moeity can affect self-aggregation which can contribute to increased half-life by slowing down biodistribution, independent of albumin5.

Following the pioneering work with insulin6, lipidation of a variety of peptides has been explored, particularly peptides within the diabetes space including human glucagon-like peptide-1 (GLP-1) analogues, glucose-dependent insulinotropic polypeptide and GLP-1 R/Glucagon receptor coagonists among others. Two lipidated peptide drugs are currently FDA-approved for use in humans. These are both long-acting anti-diabetics, the GLP- 1 analogue liraglutide and insulin detemir.

A potentially pharmacologically-relevant difference between PEGylation and lipidation is that the therapeutically active peptide is covalently linked to the much larger PEG, whereas the smaller fatty acyl-peptide conjugate is non-covalently associated with the larger

albumin, bound and unbound forms existing in equilibrium. This can result in differences in biodistribution that may result in different pharmacology as access to receptors localised in different tissues may elicit differential effects. In some cases, more restricted biodistribution may be desirable, while in others, greater tissue penetration may be important. An interesting variation of the PEG approach which addresses this issue has been developed by Santi et al in which releasable PEG conjugates with predictable cleavage rates are utilised7.

PEGylation and lipidation both confer protection against proteases and peptidases by shielding through steric hindrance and extend circulating half-life through increased hydrodynamic radius, directly or indirectly. Both methods utilise chemical conjugation and are flexible in that they are agnostic to the means used to generate the peptide they are modifying, whether biologically or synthetically produced. An advantage of using synthetic peptides is that they can incorporate non-natural amino acids designed to address a number of specific issues including instability due to known proteolytic cleavage liabilities. They can also be more flexible in terms of the choice of attachment site which is critical if activity or potency is highly dependent on the free termini or a modified residue such as a Cterminal amide.

### Classical genetic fusions: Fc and HSA

Classical genetic fusions to long-lived serum proteins offer an alternative method of half-life extension distinct from chemical conjugation to PEG or lipids. Two major proteins have traditionally been used as fusion partners: antibody Fc domains and human serum albumin (HAS). Fc fusions involve the fusion of peptides, proteins or receptor exodomains to the Fc portion of an antibody. Both Fc and albumin fusions achieve extended half-lives not only by increasing the size of the peptide drug, but both also take advantage of the body's natural recycling mechanism: the neonatal Fc receptor, FcRn. The pH-dependent binding of these proteins to FcRn prevents degradation of the fusion protein in the endosome. Fusions based on these proteins can have half-lives in the range of 3-16 days, much longer than typical PEGylated or lipidated peptides. Fusion to antibody Fc can improve the solubility and stability of the peptide or protein drug. An example of a peptide Fc fusion is dulaglutide, a GLP-1 receptor agonist currently in late-stage clinical trials. Human serum albumin, the same protein exploited by the fatty acylated peptides is the other popular fusion partner. Albiglutide is a GLP-1 receptor agonist based on this platform. A major difference between Fc and albumin is the dimeric nature of Fc versus the monomeric structure of HAS leading to presentation of a fused peptide as a dimer or a monomer depending on the choice of fusion partner. The dimeric nature of a peptide Fc fusion can produce an avidity effect if the target receptors are spaced closely enough together or are themselves dimers. This may be desirable or not depending on the target.

### Designed polypeptide fusions: XTEN and PAS

An intriguing variation of the recombinant fusion concept has been the development of designed lowcomplexity sequences as fusion partners, basically unstructured, hydrophilic amino acid polymers that are functional analogs of PEG. The inherent biodegradability of the polypeptide platform makes it attractive as a potentially more benign alternative to PEG. Another advantage is the precise molecular structure of the recombinant molecule in contrast to the polydispersity of PEG. Unlike HSA and Fc peptide fusions, in which the three-dimensional folding of the fusion partner needs to be maintained, the recombinant fusions to unstructured partners can, in many cases, be subjected to higher temperatures or harsh conditions such as HPLC purification.

The most advanced of this class of polypeptides is termed XTEN (Amunix) and is 864 amino acids long and comprised of six amino acids (A, E, G, P, S and T). Enabled by the biodegradable nature of the polymer, this is much larger than the 40 KDa PEGs typically used and confers a concomitantly greater half-life extension. The fusion of XTEN to peptide drugs results in half-life extension by 60- to 130-fold over native molecules. Two fully recombinantly produced XTENylated products have entered the clinic, namely VRS-859 (Exenatide-XTEN) and VRS- 317 (human growth hormone-XTEN). In Phase la studies, VRS-859 was found to be well-tolerated and efficacious in patients with Type 2 diabetes. VRS-317 reported superior pharmacokinetic and pharmacodynamic properties compared with previously studied rhGH products and has the potential for once-monthly dosing.

A second polymer based on similar conceptual considerations is PAS (XL-Protein GmbH)9. A random coil polymer comprised of an even more restricted set of only three small uncharged amino acids, proline, alanine and serine. Whether differences in the biophysical properties of PAS and the highly negatively charged XTEN may contribute to differences in biodistribution and/or in vivo activity is yet unknown but will be revealed as these polypeptides are incorporated into more therapeutics and the behaviour of the fusions characterised.

All the peptide protein fusions, whether the partner is Fc, HSA, XTEN or PAS, are genetically encoded and consequently suffer from similar constraints. One limitation is that only naturally occurring amino acids are incorporated, unlike the methods employing chemical conjugation which allow the use of synthetic peptides incorporating non-natural amino acids. Although methods to overcome this by expanding the genetic code are being developed by companies such as Ambrx or Sutro, they are not yet in wide use. A second limitation is that either the N- or C-terminus of the peptide needs to be fused to the partner. Oftentimes, the peptide termini are involved in receptor interactions and genetic fusion to one or both termini can greatly impair activity. Since the site of PEG or lipid conjugation can be anywhere on the peptide, it can be optimised to maximise biological activity of the resulting therapeutic.

### Hybrid methods merging synthetic peptides with half-life extension proteins

While genetic fusions have historically offered the potential for greater half-life extension, they lack the advantages afforded by the methods utilising chemical conjugation, PEGylation and lipidation, in terms of flexibility of attachment sites and incorporation of unnatural amino acids or modifications to the peptide backbone. One of the first efforts to merge the advantages of the genetic fusions with chemical conjugation for half-life extension was carried out by researchers at the Scripps Research Institute in La Jolla with the technology which later formed the basis for the biotech company CovX10,11. Using a catalytic aldolase antibody, these researchers developed a platform through which the active site lysine of the antibody forms a reversible covalent enamine bond with a betadiketone incorporated into a peptide or small molecule. The resulting complex is termed a CovXBody ^{™}. This approach combines the functional qualities of a peptide drug or small molecule with the long serum half-life of an antibody, not through a genetic fusion but rather through a chemical linkage. Following the initial demonstration of the technology, researchers expanded upon the use of CovX-Body^{™} prototype that is based on an integrin targeting peptidomimetic pharmacophore. At least three molecules based on this architecture have entered clinical development: CVX-096, a Glp-1R agonist; CVX-060, an Angiopoietin-2 binding peptide; and CVX-045, a thrombospondin mimetic.

Recently, the XTEN polypeptide has also been used in a chemical conjugation mode12 making it even more directly analogous to PEG. The first example of an XTENylated peptide that was created using this method is GLP2-2G-XTEN in which the peptide is chemically conjugated to the XTEN protein polymer using maleimide-thiol chemistry. The chemically conjugated GLP2-2GXTEN molecules exhibited comparable in vitro activity, in vitro plasma stability and pharmacokinetics in rats comparable to recombinantly-fused GLP2-2G-XTEN.

The number and spacing of reactive groups such as lysine or cysteine side chains in the completely designed sequences of XTEN or PAS polypeptides can be precisely controlled through site-directed changes due to the restricted amino acid sets from which they are composed. This provides an additional degree of flexibility over methods which might utilise Fc or albumin whose sequences naturally contain many reactive groups and stands in contrast to the CovX technology which relies on a reactive residue in a highly specialised active site. In addition, the lack of tertiary structure of XTEN or PAS should provide more flexibility over the conditions and chemistries used in coupling and in the purification of conjugates.

In summary, hybrid peptide half-life extension methods are emerging that combine the advantages and overcome the individual limitations of chemical conjugation and genetic fusions methods. These methods enable the creation of molecules based on recombinant polypeptide-based partners that impart longer half-life but free the therapeutic peptide moieties from the limitations of being composed solely of natural L-amino acids or configured solely as linear, unidirectional polypeptides fused at either the N- or C-terminus, thus opening the door to a wide range of longer acting peptide-based drugs.

"Muscle atrophy" is defined as a decrease in muscle mass and can be characterised by a complete or partial wasting of muscle, and resultant muscle weakness. It generally occurs in people that do not exercise properly, do not eat properly, or both. It is also present in subjects with muscle disease, such as myopathies (i.e. muscular dystrophy), and is a comorbidity of several diseases/conditions, including eating disorders, cancer, AIDS, COPD, ALS, physical injuries that limit exercise, degenerative conditions. It is also prevalent in elderly subjects as part of the normal ageing process.

"Elderly subject" refers to a subject that is at least 65 years old.

"Disease or condition characterised by muscle atrophy" refers to a disease or condition that includes muscle atrophy as a symptom. Examples include physical injuries (i.e. that result in the subject being physically inactive, for example muscular, nerve, bone, cartilage, ligament, disc, head or joint injuries,) diseases that cause subjects to be confined to bed or home (i.e. cancer, infectious diseases, etc), eating disorders, cachexia, sarcopaenia, malnutrition, metabolic disease (including Type I and II diabetes), and neuronal, muscle or joint degeneration diseases such as amytrophic lateral sclerosis (ALS), multiple sclerosis (MS), Parkinson's disease, Huntington's disease, muscular dystrophy, Guillane-Barre syndrome, osteoarthritis, polio, rheumatoid arthritis, spinal muscular atrophy and polymyositis. Neuronal degeneration diseases include ALS, MS, Parkinson's disease, and Huntington's disease. Muscle degeneration diseases include include myopathies (including polymyocitis), muscular dystrophy, Guillane-Barre syndrome, spinal muscular atrophy. Joint degeneration diseases include arthritic, rheumatoid arthritis, orteoarthritis,

As used herein, the term "expression vector of the invention" may be any suitable vector, including chromosomal, non-chromosomal, and synthetic nucleic acid vectors (a nucleic acid sequence comprising a suitable set of expression control elements) suitable for expression of a peptide of the invention in a cell. Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. In one embodiment, the peptide-encoding nucleic acid molecule is comprised in a naked DNA or RNA vector, including, for example, a linear expression element (as described in, for instance, Sykes and Johnston, Nat Biotech 12, 355-59 (1997)), a compacted nucleic acid vector (as described in for instance U.S. Pat. No. 6,077,835 and/or WO 00/70087), or a plasmid vector such as pBR322, pUC 19/18, or pUC 118/119. Such nucleic acid vectors and the usage thereof are well known in the art (see, for instance, U.S. Pat. No. 5,589,466 and U.S. Pat. No. 5,973,972). In one embodiment, the DNA comprises an expression control sequence.

In one embodiment, the vector is suitable for expression of the peptide of the invention in a bacterial cell. Examples of such vectors include expression vectors such as BlueScript (Stratagene), pIN vectors (Van Heeke & Schuster, 1989, J Biol Chem 264, 5503-5509), pET vectors (Novagen, Madison, Wis.) and the like. In one embodiment, the expression vector may also or alternatively be a vector suitable for expression in a yeast system. Any vector suitable for expression in a yeast system may be employed. Suitable vectors include, for example, vectors comprising constitutive or inducible promoters such as yeast alpha factor, alcohol oxidase and PGH (reviewed in: F. Ausubel et al., ed., 1987, Current Protocols in Molecular Biology, Greene Publishing and Wiley InterScience New York; and Grant et al., 1987, Methods in Enzymol 153, 516-544). In other embodiments, the expression vector is suitable for expression in baculovirus-infected insect cells. (Kost, T; and Condreay, J P, 1999, Current Opinion in Biotechnology 10 (5): 428-33.)

Expression control sequences are engineered to control and drive the transcription of genes of interest, and subsequent expression of proteins in various cell systems. Plasmids combine an expressible gene of interest with expression control sequences (i.e. expression cassettes) that comprise desirable elements such as, for example, promoters, enhancers, selectable markers, operators, etc. In an expression vector of the invention, peptide-encoding nucleic acid molecules may comprise or be associated with any suitable promoter, enhancer, selectable marker, operator, repressor protein, polyA termination sequences and other expression-facilitating elements.

"Promoter" as used herein indicates a DNA sequence sufficient to direct transcription of a DNA sequence to which it is operably linked, i.e., linked in such a way as to permit transcription of the peptide of the invention-encoding nucleotide sequence when the appropriate signals are present. The expression of a peptide-encoding nucleotide sequence may be placed under control of any promoter or enhancer element known in the art. Examples of such elements include strong expression promoters (e.g., human CMV IE promoter/enhancer or CMV major IE (CMV-MIE) promoter, as well as RSV, SV40 late promoter, SL3-3, MMTV, ubiquitin (Ubi), ubiquitin C (UbC), and HIV LTR promoters). In some embodiments, the vector comprises a promoter selected from the group consisting of SV40, CMV, CMV-IE, CMV-MIE, RSV, SL3-3, MMTV, Ubi, UbC and HIV LTR.

Nucleic acid molecules of the invention may also be operably linked to an effective poly (A) termination sequence, an origin of replication for plasmid product in E. coli, an antibiotic resistance gene as selectable marker, and/or a convenient cloning site (e.g., a polylinker). Nucleic acids may also comprise a regulatable inducible promoter (inducible, repressable, developmentally regulated) as opposed to a constitutive promoter such as CMV IE (the skilled artisan will recognize that such terms are actually descriptors of a degree of gene expression under certain conditions).

Selectable markers are elements well-known in the art. Under the selective conditions, only cells that express the appropriate selectable marker can survive. Commonly, selectable marker genes express proteins, usually enzymes,that confer resistance to various antibiotics in cell culture. In other selective conditions, cells that express a fluorescent protein marker are made visible, and are thus selectable. Embodiments include betalactamase (bla) (beta-lactam antibiotic resistance or ampicillin resistance gene or ampR), bls (blasticidin resistance acetyl transferase gene), bsd (blasticidin-S deaminase resistance gene), bsr (blasticidin-S resistance gene), Sh ble (Zeocin^{®} resistance gene), hygromycin phosphotransferase (hpt) (hygromycin resistance gene), tetM (tetracycline resistance gene or tetR), neomycin phosphotransferase II (npt) (neomycin resistance gene or neoR), kanR (kanamycin resistance gene), and pac (puromycin resistance gene).

In certain embodiments, the vector comprises one or more selectable marker genes selected from the group consisting of bla, bls, BSD, bsr, Sh ble, hpt, tetR, tetM, npt, kanR and pac. In other embodiments, the vector comprises one or more selectable marker genes encoding green fluorescent protein (GFP), enhanced green fluorescent protein (eGFP), cyano fluorescent protein (CFP), enhanced cyano fluorescent protein (eCFP), or yellow fluorescent protein (YFP).

For the purposes of this invention, gene expression in eukaryotic cells may be tightly regulated using a strong promoter that is controlled by an operator that is in turn regulated by a regulatory protein, which may be a recombinant "regulatory fusion protein" (RFP). The RFP consists essentially of a transcription blocking domain, and a ligand-binding domain that regulates its activity. Examples of such expression systems are described in US20090162901A1, which is herein incorporated by reference in its entirety.

As used herein "operator" indicates a DNA sequence that is introduced in or near a gene in such a way that the gene may be regulated by the binding of the RFP to the operator and, as a result, prevents or allow transcription of the gene of interest, i.e. a nucleotide encoding a peptide of the invention. A number of operators in prokaryotic cells and bacteriophage have been well characterized (Neidhardt, ed., Escherichia coli and Salmonella; Cellular and Molecular Biology 2d. Vol 2 ASM Press, Washington D.C. 1996). These include, but are not limited to, the operator region of the LexA gene of E. coli, which binds the LexA peptide, and the lactose and tryptophan operators, which bind the repressor proteins encoded by the Lad and trpR genes of E. coli. These also include the bacteriophage operators from the lambda PR and the phage P22 ant/mnt genes, which bind the repressor proteins encoded by lambda cl and P22 arc. In some embodiments, when the transcription blocking domain of the RFP is a restriction enzyme, such as Notl, the operator is the recognition sequence for that enzyme. One skilled in the art will recognize that the operator must be located adjacent to, or 3' to the promoter such that it is capable of controlling transcription by the promoter. For example, U.S. Pat. No. 5,972,650, which is incorporated by reference herein, specifies that tetO sequences be within a specific distance from the TATA box. In specific embodiments, the operator is preferably placed immediately downstream of the promoter. In other embodiments, the operator is placed within 10 base pairs of the promoter.

In an exemplary cell expression system, cells are engineered to express the tetracycline repressor protein (TetR) and a protein of interest is placed under transcriptional control of a promoter whose activity is regulated by TetR. Two tandem TetR operators (tetO) are placed immediately downstream of a CMV-MIE promoter/enhancer in the vector. Transcription of the gene encoding the protein of interest directed by the CMV-MIE promoter in such vector may be blocked by TetR in the absence of tetracycline or some other suitable inducer (e.g. doxycycline). In the presence of an inducer, TetR protein is incapable of binding tetO, hence transcription then translation (expression) of the protein of interest occurs. (See, e.g., U.S. Pat. No. 7,435,553, which is herein incorporated by reference in its entirety.)

The vectors of the invention may also employ Cre-lox recombination tools to facilitate the integration of a gene of interest into a host genome. A Cre-lox strategy requires at least two components: 1) Cre recombinase, an enzyme that catalyzes recombination between two IoxP sites; and 2) IoxP sites (e.g. a specific 34-base pair by sequence consisting of an 8-bp core sequence, where recombination takes place, and two flanking 13-bp inverted repeats) or mutant lox sites. (See, e.g. Araki et al., 1995, PNAS 92:160-4; Nagy, A. et al., 2000, Genesis 26:99-109; Araki et al., 2002, Nuc Acids Res 30(19):e103; and US20100291626A1, all of which are herein incorporated by reference). In another recombination strategy, yeast-derived FLP recombinase may be utilized with the consensus sequence FRT (see also, e.g. Dymecki, S. M., 1996, PNAS 93(12): 6191-6196).

As used herein, the term "host cell" includes any cell that is suitable for expressing a recombinant nucleic acid sequence. Cells include those of prokaryotes and eukaryotes (single-cell or multiple-cell), bacterial cells (e.g., strains of E. coli, Bacillus spp., Streptomyces spp., etc.), mycobacteria cells, fungal cells, yeast cells (e.g. S. cerevisiae, S. pombe, P. partoris, P. methanolica, etc.), plant cells, insect cells (e.g. SF-9, SF-21, baculovirus-infected insect cells, Trichoplusia ni, etc.), non-human animal cells, mammalian cells, human cells, or cell fusions such as, for example, hybridomas or quadromas. In certain embodiments, the cell is a human, monkey, ape, hamster, rat or mouse cell. In other embodiments, the cell is eukaryotic and is selected from the following cells: CHO (e.g. CHO K1, DXB-11 CHO, Veggie-CHO), COS (e.g. COS-7), retinal cells, Vero, CV1, kidney (e.g. HEK293, 293 EBNA, MSR 293, MDCK, HaK, BHK21), HeLa, HepG2, WI38, MRC 5, Colo25, HB 8065, HL-60, Jurkat, Daudi, A431 (epidermal), CV-1, U937, 3T3, L cell, C127 cell, SP2/0, NS-0, MMT cell, tumor cell, and a cell line derived from an aforementioned cell. In some embodiments, the cell comprises one or more viral genes, e.g. a retinal cell that expresses a viral gene (e.g. a PER.C6^{®} cell). In some embodiments, the cell is a CHO cell. In other embodiments, the cell is a CHO K1 cell.

As used herein, the term "transformed cell of the invention" refers to a host cell comprising a nucleic acid stably integrated into the cellular genome that comprises a nucleotide sequence coding for expression of a peptide of the invention. In another embodiment, the present invention provides a cell comprising a non-integrated (i.e., episomal) nucleic acid, such as a plasmid, cosmid, phagemid, or linear expression element, which comprises a sequence coding for expression of a peptide of the invention. In other embodiments, the present invention provides a cell line produced by stably transfecting a host cell with a plasmid comprising an expression vector of the invention.

As used herein, the term "engineered" as applied to a cell means genetically engineered using recombinant DNA technology, and generally involves the step of synthesis of a suitable expression vector (see above) and then transfecting the expression vector into a host cell (generally stable transfection).

As used herein, the term "heterologous expression" refers to expression of a nucleic acid in a host cell that does not naturally have the nucleic acid. Insertion of the nucleic acid into the heterologous host is performed by recombinant DNA technology.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

### Materials and Methods

### CELL CULTURE ASSAY PREPARATION: C2C12 preparation

### Growth Medium Preparation:

To 500mL of 4.5g/L glucose DMEM add 5mL of L-Glutamine solution (Final concentration: 1%), 5mL Penicillin-Streptomycin (Final concentration: 1%) and 50mL of sterile filtered foetal bovine serum previously heated at 55oC for 30minutes (Final concentration: 10%).

### Differentiation Medium Preparation:

To 500mL of 4.5g/L glucose DMEM add 5mL of L-Glutamine solution (Final concentration: 1%), 5mL Penicillin-Streptomycin (Final concentration: 1%) and 10mL of heat inactivated horse serum (Final concentration: 2%).

### Starvation Medium Preparation:

To 500mL of 4.5g/L glucose DMEM add 5mL of L-Glutamine solution (Final concentration: 1%) and 5mL Penicillin-Streptomycin (Final concentration: 1%).

### S6 PHOSPHORYLATION ASSAY WORKFLOW

*Day 1:* In a 96-well plate, seed 2400 cells/well (8,000cells/cm2) in 100 ul/well of Growth Medium. Let them adhere and grow for 48h at 37°C, 5% CO2. (See Thawing subculturing C2C12 SOP 58 for more details).

*Day 3:* Remove Growth medium and add 100ul/well of Differentiation medium. Let them differentiate for 7 days at 37°C, 5% CO2, by adding fresh differentiation medium every day if possible. (See Differentiation of C2C12 SOP60 for more details).

*Day 8:* Remove differentiation media and add 100ul/well of Starvation medium to starve the cells for 3h at 37°C, 5% CO2 After that, remove Starvation medium and add 100ul/well of HBSS and incubate at 37°C, 5% CO2 to deprive cell of amino acids for 1h.

Make up the peptide and hydrolysate treatments in 2ml test tubes by diluting the peptides/hydrolysates in HBSS to make up the desired concentration (typically 0.5ug/ml and 5ug/ml). You need a minimum volume of 300ul to treat 3 wells (100ul/well). Use an Insulin treatment at 0.1uM as a positive control. All treatments should be completed in triplicates. The treatment time recommended is 30 minutes.

### CELL CULTURE ASSAY PREPARATION:THP-1 Cell Culture

Human monocytic leukaemia (THP-1) cells (ECACC collection; Sigma-Aldrich, St Louis, MO, USA) were maintained in culture in the Roswell Park Memorial Institute medium (RPMI 1640, Lonza, Basel, Switzerland) supplemented with 1% L-glutamine, 10% heatinactivated FBS, 1% penicillin-streptomycin, and a 10% sterile filtered foetal bovine serum previously heated at 55 °C for 30 min.

### TNF-A SECRETION ASSAY WORKFLOW

THP-1 derived TNF released into the supernatants was assessed by using the TNF-α ELISA kit (BioLegend, San Diego, California, USA) according to the manufacturer's instructions. To differentiate into macrophages, THP-1 cells were seeded (2 × 10⁶ well⁻¹) in 6-well plates and treated with a 100 nM phorbol-12-myristate-13-acetate (PMA; Sigma-Aldrich, St Louis, MO, USA) for 72 h at 37 °C, 5% CO₂. After incubation, non-attached cells were aspirated, and adherent cells were treated with NPN_1 (0.5-5 µg/mL) in duplicate or triplicate. Following incubation for 24 h, lipopolysaccharide (LPS) from Escherichia coli O127:B8 (Sigma-Aldrich, St Louis, MO, USA) was added to 100 ng/mL for 24 h at 37 °C, 5% CO₂. Cell supernatants were collected, and THP-1 derived TNF released into supernatants was assessed by using the TNF-α ELISA kit (BioLegend, San Diego, California, USA) according to the manufacturer's instructions.

### RNA ISOLATION FROM C2C12 CELLS AND REAL-TIME QPCR

C2C12 cells were plated in 6-well plates and left to grow and differentiate at 37 °C, 5% CO₂. The cells were subsequently starved for 24 h in a starvation media at 37 °C, 5% CO₂. As per Menconi et al. (2008), to induce atrophy the cells were treated with 100 µM dexamethasone solubilized in a DMEM-LM (30030, BIOSCIENCES) supplemented 1% penicillin-streptomycin for 24 h at 37 °C, 5% CO₂[33]. Thirty minutes prior to the end of the atrophy induction, the cells were treated with NPN added on top of the dexamethasone treatment and incubated for 30 min at 37 °C, 5% CO₂. Dilutions were calculated to get the desired concentration with the final volume of 2 mL/well. An equal volume of the treatment added to each well was first removed from the dexamethasone treatment without disturbing the cells. C2C12 cells were lysed with TRIzol (Invitrogen, Carlsbad, USA), and total RNA was extracted using the Purelink RNA mini kit (Invitrogen by Thermo Fisher Scientific) according to the manufacturer's instructions. Total RNA (1 µg) was reverse transcribed to cDNA using the high-capacity cDNA reverse transcription kit (Thermo Fisher, Waltham, MA, USA). The quantitative PCR was performed using the TaqMan probe-based method, where mRNA expression was detected using a TaqMan fluorogenic gene expression probe (ABI Biosystems, CA, USA) for Trim63 (Mm01185221_m1) and Fbxo32 (Mm00499518_m1) for the experiment using C2C12. A master mix containing primer/probe and TaqMan^{®} gene expression master mix (ABI Biosystems, CA, USA) was added to 1 µL cDNA template. A final volume of 9 µL was pipetted, in duplicate, on a Roche Optical 96-well reaction plate and real-time PCR was performed on a Roche lightcycler 480 real-time PCR instrument. The threshold cycle (Ct) for each well was calculated using the instrument software. Data analysis was based on the ΔΔCt method with raw data normalized by the B2M housekeeping gene (Mm00437762_m1) included on the plate. All gene expression was compared to dexamethasone, as dexamethasone was used to induce atrophy in C2C12 cells. NPN_1 was added subsequently to examine if it could attenuate the atrophy effect caused by the dexamethasone. Results are expressed as fold over control.

### DISUSE MURINE ATROPHY STUDY PROTOCOL

This study was carried out with Melior Discovery, USA. Twelve-week old male C57b1/6J mice (N = 10/group) were randomly assigned to treatment groups based on bodyweight (10 days post-ring implantation). Ethical approval was granted by the International Association of Religious Freedom (IARF #:MLR-I15) and therefore, been performed in accordance with the ethical standards laid down by the Institutional Animal Care and Use Committee (IACUC).

The study consisted of five treatment groups (1) Healthy control (control weight bearing), (2) hindlimb unloaded (HU)-control vehicle (atrophy), (3) Bowman-Birk inhibitor (BBI; 113.3 mg/kg positive control), (4) casein (650 mg/kg; positive control), (5) NPN_1 (650 mg/kg). Briefly, prior to hindlimb unloading a tail ring was formed with a 2-0 sterile surgical steel wire that was passed through the 5th, 6th, or 7th inter-vertebral disc space and shaped into a ring from which the mice were suspended. The vertebral location for the tail-ring was selected to appropriately balance the animal body weight without interfering with defecation. The animals were suspended by a swivel harness attached at the top of the cage. The body of the animal was maintained at a 30° elevation such that only the forelimbs are to maintain contact with the cage floor. The animal could move and freely access food and water within the cage during this procedure. The height of the animal was checked daily and adjusted if necessary [34]. Mice were given seven days to acclimatize and condition, followed by 10-13 days of recovery time after tail-ring implantation, based on the IACUC guideline. The primary endpoint of this study was to assess the wet weight of the soleus muscle contained within the hindlimb of control and test mice directly after 19 days of hindlimb suspension. In addition, fixed muscle samples (5/group) were sent to CaresBio Laboratory LLC for immunofluorescence (IF) staining of Type I and Type Ila muscle fibre markers and image analysis. Soleus muscle tissue samples (10/group) were also sent to Cellomatics Biosciences LTD for gene expression analysis.

### DOSING AND MUSCLE WET MASS

All mice were dosed with either NPN_1, BBI or Casein from day 1 to day 18. On day 19, animals were sacrificed by cervical dislocation; blood/plasma samples were collected, the soleus muscles were isolated and weighed using a digital platform balance. The wet muscle weights were normalized to body weights (mg/g). One side of the soleus muscle was snap frozen and the other side of the soleus muscle was fixed in 4% fresh PBS-buffered formaldehyde.

### IMMUNOFLUORESCENCE ANALYSIS OF SOLEUS

The collected soleus muscles were post-fixed in 4% fresh PBS-buffered formaldehyde. Five samples from each of the treatment groups were randomly selected for immunofluorescence analysis. Muscles were paraffin-embedded and sectioned. Immunofluorescence labeling was used to stain Type I and Type Ila muscle fibres. Image analysis was performed on representative regions of each sample for both staining labels. As samples were processed, sections (thickness, 8 µM) were cut and immunofluorescence staining was performed as previously described [35]. Briefly, slides were subjected to heat induced antigen retrieval in a citrate buffer (10 mM, pH 6.0) and were incubated overnight with primary antibodies ab11083 (dilutions 1:250) and ab91506 (dilutions 1:200), after blocking with a nonspecific antigenicity blocker. Both of the primary antibody concentrations were determined after optimization in the test slides. Corresponding fluorescent conjugated secondary antibodies (Alexa 594 and Alexa 488) were applied for 1 h at room temperature. 4,6-Diamidino-2-phenylindole (DAPI) were included with the secondary antibodies to visualize the nuclei.

### IMAGE ACQUISITION AND ANALYSIS

The slides were scanned using a customized, computer-controlled microscope (with xystage and z controller, a Zeiss microscope, Carl Zeiss GMBh, Jena, Germany) with X4, and X10 objectives. Images were analyzed using an image analysis software based on MATLAB (R2011b, MathWorks). The baseline for the scanning setup was done using the HU-control group. Image analysis algorithms were applied to the images generated from microscopic slides of tissues stained with secondary antibody controls to generate the background score. The control/baseline was used to generate the algorithm to differentiate between the signals and signal-to-noise ratio which was applied to all images. Each marker was quantified by the single channel-based analysis. Automatic background subtraction was performed. Intensity scores for all the markers were then calculated that correspond to the average signal intensity divided by a locale area. Significant differences in relative areas stained and mean specific intensity for the stains of different groups in mouse muscle tissue were calculated. Raw data is presented, and no normalization was performed.

### RNA ISOLATION FROM MOUSE SOLEUS TISSUE AND GENE ARRAYS

Gene expression analysis was performed on soleus tissue samples. 300 µL of a homogenization solution with 3 µl Proteinase K (Quantigene Plex Assay, Invitrogen) were added to 10 mg of frozen tissue to prepare concentrated lysates. One 5-mm stainless steel bead was added to the tubes and placed in a Bullet Blender^{®} homogenizer. The tissue was then homogenized for 2 min at Speed 10. The tubes were allowed to cool at room temperature and the process was repeated until no visible particles remained. The tissue lysates were then incubated at 65 °C for 30 min, followed by centrifugation at 16,000× *g* for 15 min and the supernatant was used immediately for the assay kit (as per the manufacturer's guidelines). The net mean fluorescent intensity (MFI) was obtained from the Luminex for all the genes.

**Table 1. Fold regulated gene expression following NPN_1 treatment. RNA was extracted from collected soleus tissue samples from control vehicle and NPN_1 treated animals (N=10/group). Genes related to myogenesis and mitochondrial biogenesis were upregulated.**

| **Gene** | **Fold Change** | **Gene** | **Fold Change** |
|---|---|---|---|
| **mTOR** | 4.19 | | |
| **ESRRA** | 3.15 | | |
| **MYFS** | 3.06 | | |
| **TFAM** | 3.44 | | |
| **WNT1** | | -3.17 | |
| **IGF1R** | | -3.42 | |
| **MYC** | | -3.44 | |
| **CPT1B** | | | NC |
| **NRF1** | | | NC |
| **NRF2** | | | NC |
| **KRAS** | | | NC |

### Compositions

### Food supplement powder I

| Ingredient | Amount (g) | Amount (%) |
|---|---|---|
| Sodium caseinate | 999.52 | 99.952 |
| Peptide of SEQ ID 1 | 0.48 | 0.048 |

### Food supplement powder II

| Ingredient | | Amount (g) | Amount (%) |
|---|---|---|---|
| Sodium caseinate | | 999.74 | 999.74 |
| Peptide of SEQ ID 3 | | 0.26 | 0.026 |

### Food supplement powder III

| Ingredient | Amount (g) | Amount (%) |
|---|---|---|
| Sodium caseinate | 999.96 | 99.996 |
| Peptide of SEQ ID 2 | 0.48 | 0.048 |

### Food supplement powder IV

| Ingredient | Amount (g) | Amount (%) |
|---|---|---|
| Sodium caseinate | 999.99 | 99.999 |
| Peptide of SEQ ID 8 | 0.01 | 0.001 |

### Food supplement powder V

| Ingredient | Amount (g) | Amount (%) |
|---|---|---|
| Sodium caseinate | 999.62 | 999.62 |
| Peptide of SEQ ID 11 | 0.38 | 0.38 |

### Food supplement powder VI

| Ingredient | Amount (g) | Amount (%) |
|---|---|---|
| Sodium caseinate | 999.26 | 99.926 |
| Peptide of SEQ ID 3 | 0.48 | 0.048 |
| Peptide of SEQ ID 8 | 0.26 | 0.026 |

### Food supplement powder VII

| Ingredient | Amount (g) | Amount (%) |
|---|---|---|
| Sodium caseinate | 998.73 | 99.873 |
| Peptide of SEQ ID 3 | 0.30 | 0.03 |
| Peptide of SEQ ID 8 | 0.04 | 0.004 |
| Peptide of SEQ ID 11 | 0.53 | 0.053 |
| Peptide of SEQ ID 10 | 0.01 | 0.001 |
| Peptide of SEQ ID 19 | 0.39 | 0.039 |

### Topical Composition

| Ingredient | Exemplary (w/w)% |
|---|---|
| Phase A | |
| Water | 73.95 |
| Carbomer | 0.15 |
| Phase B | |
| Glycerin | 3.5 |
| Phase C | |
| Steareth 2 | 0.40 |
| Cetearyl alcohol dicetyl phosphate and Ceteth 10 phosphate | 4 |
| Cyclohexsiloxane | 2 |
| Dioctyl succinate | 7 |
| Steareth 10 | 1.2 |
| Mixed parabens | 0.30 |
| Phase D | |
| Sorbate | 0.10 |
| Phase E | |
| Water | 2.50 |
| Sodium hydroxide | 0.30 |
| Phase F | |
| Fragrance | 0.10 |
| Phase G | |
| Peptide(s) (One or more of SEQ ID 1-19) | 0.1 to 0.0001 |

The topical composition may be applied topically to a subject suffering from muscle atrophy, or to a healthy person after strenuous physical activity.

Percentages are examples only and it will be appreciated that any suitable percentage may be used depending on the use.

The emulsion is prepared in the following way: Phase A: disperse Ultrez 10 (carbomer) in water and let is swell for 20 minutes, then add phase B; heat to 75°C. Heat Phase C separately to 75°C. Mix the two phases under stirring, homogenise, add Phase D, neutralise with Phase E, cool until reaching 30°C, then add Phase F and Phase G; adjust to pH to 6 with ~NaOH. It will be understood that this is an example only and any suitable method known in the art may be used.

### Equivalents

The foregoing description details presently preferred embodiments of the present invention. Numerous modifications and variations in practice thereof are expected to occur to those skilled in the art upon consideration of these descriptions. Those modifications and variations are intended to be encompassed within the claims appended hereto.

## Claims

1. A composition comprising a peptide having up to 50 amino acids and comprising a sequence selected from SEQ ID 1 to SEQ ID 20.

2. A composition according to Claim 1, in which the peptide has 6 to 20 amino acids.

3. A composition according to Claim 1 or 2, in which the peptide comprises a sequence selected from SEQ ID 1 and SEQ ID 8.

4. A composition according to Claim 4, in which the peptide comprising a sequence of SEQ ID 8 is selected from SEQ ID 8 and SEQ ID 7.

5. A composition according to any preceding Claim, in which the composition is a comestible powder.

6. A method of promoting muscle synthesis in a healthy subject, comprising a step of orally administering a therapeutically effective amount of a composition of any of Claims 1 to 6 to the healthy subject.

7. A method of inhibiting muscle loss in a healthy subject, comprising a step of orally administering a therapeutically effective amount of a composition of any of Claims 1 to 5 to the healthy subject.

8. A method of improving an inflammatory response in a healthy subject, comprising a step of orally administering a therapeutically effective amount of a composition of any of Claims 1 to 5 to the healthy subject.

9. A composition of any of Claims 1 to 5, for use in a method of preventing or treating muscle atrophy in a subject.

10. An isolated peptide having up to 50 amino acids and comprising SEQ ID 1, SEQ ID 7 or SEQ ID 8.

11. An isolated peptide according to Claim 10, having 6-20 amino acids.

12. Use of an isolated peptide according to Claim 10 or 11, or a composition according to any of Claims 1 to 5, to promote muscle synthesis in a healthy subject.

13. An isolated nucleic acid encoding a peptide of Claim 10 or 11.
